(19) **Europäisches Patentamt / European Patent Office / Office européen des brevets**

(11) **EP 1 839 642 B1**

(12) **EUROPEAN PATENT SPECIFICATION**

(45) Date of publication and mention
of the grant of the patent:
**11.04.2012 Bulletin 2012/15**

(51) Int Cl.:
*A61K 8/02* (2006.01)    *D04H 13/00* (2006.01)
*B32B 5/26* (2006.01)

(21) Application number: **07004063.9**

(22) Date of filing: **27.02.2007**

(54) **Moisturized nonwoven fabric**

Befeuchtetes Vlies

Étoffe non tissée humidifiée

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.03.2006 JP 2006094451**

(43) Date of publication of application:
**03.10.2007 Bulletin 2007/40**

(73) Proprietor: **Kawano Paper Co., Ltd.
Kochi-shi
Kochi 780-0934 (JP)**

(72) Inventors:
• **Suzuki, Shinji
Kochi Prefectural Paper Tech. Center,
Kochi 781-2128 (JP)**
• **Ike, Noriyasu
Kochi Prefectural Paper Tech. Center,
Kochi 781-2128 (JP)**
• **Sawamura, Kiyotsugu
Kochi Prefectural Paper Tech. Center,
Kochi 781-2128 (JP)**
• **Matsumoto, Hiromu
Kochi Prefectural Paper Tech. Center,
Kochi 781-2128 (JP)**
• **Tamura, Eri
Kochi Prefectural Paper Tech. Center,
Kochi 781-2128 (JP)**
• **Morisawa, Jun
Kochi Prefectural Paper Tech. Center,
Kochi 781-2128 (JP)**

• **Takiguchi, Hiroto
Kochi Prefectural Paper Tech. Center,
Kochi 781-2128 (JP)**
• **Ariyoshi, Masaaki
Kochi Prefectural Paper Tech. Center,
Kochi 781-2128 (JP)**
• **Taniguchi, Kenji
Kawano Paper Co., Ltd Saitama-Mill
Saitama 349-0135 (JP)**
• **Yoshida, Hideaki
Kawano Paper Co., Ltd Saitama-Mill
Saitama 349-0135 (JP)**
• **Tsuruta, Hitoshi
Sanshoshigyo Co., Ltd. Fushokufu-Mill,
Kochi 781-1101 (JP)**
• **Sasaoka, Masaaki
Sanshoshigyo Co., Ltd.
Kochi 781-1111 (JP)**
• **Sasaki, Akihiro
Sanshoshigyo Co., Ltd.
Kochi 781-1111 (JP)**

(74) Representative: **Koepe, Gerd L.
Koepe & Partner
Robert-Koch-Strasse 1
80538 München (DE)**

(56) References cited:
EP-A- 1 306 073      EP-A2- 1 222 915
WO-A-00/64408       US-A1- 2003 049 290
US-A1- 2003 114 324   US-A1- 2003 119 395

## Description

BACKGROUND OF THE INVENTION

1. Field of the Invention

The invention relates to a moisturized nonwoven fabric provided with a moisture retention property by adding a moisture-retaining component, more particularly a moisturized nonwoven fabric for wiping use, nursing care use, or cosmetic use and is made preferably usable for keeping skin moisture and wiping for dry skin or in a dry season such as winter by improving the water absorption property, water retention property, wiping property, and skin touch.

2. Description of the Related Art

Conventionally, wet tissues obtained by impregnating nonwoven fabrics with about 200 to 500% of liquids containing water or alcohol as main components have been made available (JP-A No. 2005-287710) as well as WO-A 00/64,408; EP-A 1 222 915; US-A 2003/0,114,324; and US-A 2003/0.049,290.
Further, moisturized tissues obtained by impregnating tissues with various kinds of moisture-retaining components have been made available (JP-A No. 5-156596).
As a conventional drylaid nonwoven fabric, spunlaced nonwoven fabrics for wiping which contain cellulose fibers such as cotton and thermoplastic fibers and are made soft by controlling the fiber density have been made available (Japanese Patent No. 3578859).
The document EP-A 1 306 073 relates to a fibrous web product in which a water-containing gel composition is contained in a fibrous web, wherein the water-containing gel composition contains a moisture retaining component, a gelling agent and water, and the water is at least one of water retained by the moisture retaining component and water absorbed from the atmosphere by the moisture retaining component.

SUMMARY OF THE INVENTION

However, the conventional wet tissues have problems: 1) cool at the time of use; 2) irritating the skin; 3) inevitably requiring a preservative; and 4) needing a wrapping container for prevention of drying, and in the case of use for babies and elders, who are weak against skin irritation, these tissues have sometimes caused skin troubles.
Further, the conventional moisturized tissues have problems: 1) low strength; 2) small water retention property; and 3) the strength is sacrificed if the impregnation amount of the moisture-retaining component is increased to improve moisture retention effect on the skin, and these tissues have been limited in use only for wiping at the time of blowing one's nose.
Although the conventional drylaid nonwoven fabrics are improved more or less in the softness by improving the production process, they are insufficient in smoothness, softness, and moist feeling.
Accordingly, the invention aims to solve the problems which the conventional wet tissues and moisturized tissues have and provide a moisturized nonwoven fabric excellent in use feel such as skin touch and wiping property and functionalities such as water absorption property and water retention property. Further, the invention aims to provide a moisturized nonwoven fabric excellent in functionalities such as high mobility of moisturizing liquid to the skin and skin moisturizing effect.
The present invention relates to a moisturized non-woven fabric made by applying a moisturizing liquid including a water-soluble component, containing one or more moisture-retaining component(s), selected from glycerin, diglycerin, polyglycerin, ethylene glycol, diethylene glycol, polyethylene glycol, propylene glycol, 1,3-butylene glycol, sorbitol, xylitol, erythritol, mannitol, lactitol, oligosaccharide alcohol, maltitol, reduced starch hydrolysis product, fructose, glucose, oligosaccharide, trehalose, glycine betaine, pyrrolidonecarboxylic acid, pyrrolidone carboxylic acid salt, hyaluronic acid, hyaluronic acid salt, lactic acid, lactic acid salt, and urea, and water to a raw non-woven fabric through a moisturizing treatment, wherein the raw non-woven fabric comprises 20 to 80 % by weight of fibers with a length of 20 to 100 mm and a fineness of 0.5 to 10.0 dtex and 20 to 80 % by weight of natural cellulose fibers with a length shorter than 20 mm and is spunlaced, wherein the amount of the water-soluble component is 5 to 150 % to the weight of the raw non-woven fabric; and wherein the increase ratio of the equilibrium moisture regain of the moisturized non-woven fabric is 0.5 to 40 % as compared with an equilibrium moisture regain of the moisturized non-woven fabric after the water soluble component is removed, measured according to the procedure of JIS P8127 (Paper and board - Determination of moisture content - Oven-drying method).
The moisturized nonwoven fabric of the invention contains 1% or more of a water-soluble component to a weight of a pre-moisturized nonwoven fabric and an increase ratio of an equilibrium moisture regain (equilibrium water content) is 0.5% or more as compared with an equilibrium moisture regain of the moisturized nonwoven fabric after the water-soluble component is removed. The moisturized nonwoven fabric means a nonwoven fabric which contains a moisture-retaining component. The water-soluble component is a component which is washed away when the moisturized nonwoven fabric is washed with distilled water at 60°C (excluding depleted fibers). The water-soluble component contains the moisture-retaining component, water which is retained by the moisture-retaining component when the moisture-retaining component is in an equilibrium state with the atmospheric air, and a water-soluble component or a water dispersible component which is obtained by, for example, solubilizing or emulsifying a lipophilic substance with a surfactant.

The content of the water-soluble component is 1 to 250%, preferably 5 to 150%, and more preferably 10 to 100%. Content of the water-soluble component can be controlled by the coating amount of the moisturizing liquid to be applied to a pre-moisturized nonwoven fabric (a raw nonwoven fabric). If the content of the water-soluble component is less than 1%, the nonwoven fabric is felt stiff. If it is higher than 250%, it gives sticky feeling. In the case the moisturized nonwoven fabric is ' required to have soft, non-sticky and smooth skin touch, the content is most preferably 10 to 50%. In the case the moisturized nonwoven fabric is required to have moist feeling and moisturizing effect on the skin, the content is most preferably 50 to 100%.

Increase ratio of an equilibrium moisture regain of the moisturized nonwoven fabric is preferably 0.5 to 40% and more preferably 1.0 to 20% as compared with an equilibrium moisture regain of the moisturized nonwoven fabric after the water-soluble component is removed. In the case the moisturized nonwoven fabric is required to have soft, fresh and smooth skin touch, the increase ratio is most preferably 1.0 to 3.0%. In the case the moisturized nonwoven fabric is required to have moist feeling and moisturizing effect on the skin, the increase ratio is most preferably 3.0 to 10%. If the increase ratio of the equilibrium moisture regain is 0.5% or lower, the moisturized nonwoven fabric becomes insufficient to exhibit the properties such as softness and moisture feeling. If the increase ratio of the equilibrium moisture regain is 40% of higher, sticky feeling is intensified and thus it is not preferable.

The moisturized nonwoven fabric of the invention may have F5-value (tensile strength measured at an elongation of 5%) per unit METSUKE (weight per unit surface area) of 0.40 N·m$^2$/g or lower in the strongest direction and the strength of 1.0 N or more in the weakest direction. The moisturized nonwoven fabric satisfying the above-mentioned conditions has soft touch and good feeling since it can be deformed and fit on hands at the time of use and is provided with sufficient strength for use as well.

F5-value per unit METSUKE in the strongest direction is 0.40 N·m$^2$/g or lower, preferably 0.20 N·m$^2$/g or lower, and more preferably 0.15 N·m$^2$/g or lower. If F5-value per unit METSUKE in the strongest direction is 0.40 N·m$^2$/g or higher, the moisturized nonwoven fabric is felt hard and stiff and thus uncomfortable to use.

The strength in the weakest direction is 1.0 N or higher, preferably 2.0 N or higher, and more preferably 3.0 N or higher. Unless the strength in the weakest direction is 1.0 N or higher, the moisturized nonwoven fabric may be torn during use and is thus inconvenient to use.

The moisturized nonwoven fabric of the invention may have the water absorption capacity per 1 g of the moisturized nonwoven fabric in a range from 0.03 to 2.50 ml/g after 5 seconds from the starting of measurement by Larose method and water retention ratio of 3.0 or higher. If the wet skin is wiped with the moisturized nonwoven fabric satisfying the above-mentioned conditions, the moisture-retaining component contained in the moisturized nonwoven fabric is eluted due to the water remaining on the skin so that a proper amount of the moisture-retaining component is left on the skin together with the water and accordingly the skin strongly feels moisture.

The water absorption capacity per 1 g of the moisturized nonwoven fabric is in a range from 0.03 to 2.50 ml/g after 5 seconds from the starting of measurement by Larose method. In the case the moisture retention property is emphasized for the skin during use, it is preferably in a range from 0.03 to 0.30 ml/g and in the case the water absorption property is emphasized during use, it is preferably in a range from 0.30 to 2.50 ml/g. If the water absorption capacity is 0.03 ml/g or lower, water cannot be wiped completely when the wet skin is wiped with the nonwoven fabric and thus the use feel is inferior. If the water absorption capacity is 2.50 ml/g or higher, the wiping is carried out too extremely to transfer the moisture-retaining component of the moisturized nonwoven fabric to the skin and thus the nonwoven fabric cannot give a moist feeling to the skin.

The water retention ratio is preferably 3.0 or higher and more preferably 4.0 or higher. If the water retention ratio is 3.0 or lower, the water component is soon saturated when the nonwoven fabric is used for wiping the wet skin and the nonwoven fabric cannot be used as a wiping sheet.

The moisturized nonwoven fabric of the invention may have a bending resistance of 5.0 mN·m$^2$/g or lower per unit METSUKE in the highest bending resistance direction measured by a handle O meter. The bending resistance per unit METSUKE may be 1.5 mm·m$^2$/g or lower in the highest bending resistance direction measured by a cantilever method. The moisturized nonwoven fabric which satisfies these conditions is soft and easily bent and gives a good drape property.

The bending resistance per unit METSUKE in the highest bending resistance direction is 5.0 mN·m$^2$/g or lower, preferably 4.0 mN·m$^2$/g or lower measured by the handle O meter. The bending resistance per unit METSUKE is preferably 1.5 mm·m$^2$/g or lower in the highest bending resistance direction measured by a cantilever method. The moisturized nonwoven fabric which does not satisfy these conditions is hard to be bent and gives stiff feeling at the time of use.

The drape coefficient is 68% or lower, preferably 65% or lower, and more preferably 60% or lower. If the drape coefficient is 68% or lower, the moisturized nonwoven fabric is soft and compatible with the skin, and is easy to be deformed along the hands of a user when used as a wiper, and thus preferable to wipe delicate portions.

The KES bending rigidity B value is 0.20 $gf/cm^2/cm$ or lower, preferably 0.15 $gf/cm^2/cm$ or lower, and more preferably 0.10 $gf/cm^2/cm$ or lower, in the direction in which the B value is highest. If KES bending rigidity B value is 0.20 $gf/cm^2/cm$ or lower in the direction in which the B value is highest, the moisturized nonwoven fabric is felt soft and easy to be bent. Accordingly, in the case the nonwoven fabric is used as a wiper, it scarcely gives stiff feeling.

The KES shear stiffness G value is 2.00 g/cm/degree or lower, preferably 1.50 g/cm/degree or lower, and more preferably 1.30 g/cm/degree or lower, in the direction in which the G value is highest. The KES shear rigidity G value is a value indicating the amount of stress when a shear force is applied to the nonwoven fabric. When the G value is small, it means that the nonwoven fabric is soft and easy to be bent, and compatible with the skin. If KES shear rigidity G value is 2.00 g/cm/degree or lower in the direction in which the G value is highest, the moisturized nonwoven fabric is felt soft, and accordingly, in the case the nonwoven fabric is used as a wiper, it scarcely gives stiff feeling.

The moisturized nonwoven fabric of the invention may have 0.45 or lower MIU value (friction coefficient) by the friction tester. When the moisturized nonwoven fabric satisfying this condition is touched, it gives smooth feeling. The moisturized nonwoven fabric having 0.45 or higher MIU value scarcely gives smooth feeling when its surface is touched.

The moisturized nonwoven fabric of the invention may have the peak value of the heat flow Qmax in a range from 0.08 to 0.30 $J/cm^2/sec$.

The peak value of the heat flow Qmax is preferably in a range from 0.09 to 0.20 $J/cm^2/sec$. In the case the moisturized nonwoven fabric is required to have soft, non-sticky and smooth skin touch, it is most preferably in a range from 0.09 to 0.115 $J/cm^2/sec$. In the case the moisturized nonwoven fabric is required to have moist feeling and moisturizing effect on the skin, it is most preferably in a range from 0.115 to 0.20 $J/cm^2/sec$. If Qmax is 0.08 $J/cm^2/sec$ or lower, the moisturized nonwoven fabric scarcely gives moist feeling. If Qmax is 0.30 $J/cm^2/sec$ or higher, the moisturized nonwoven fabric is felt cold when it is touched.

[Moisturizing treatment]

The moisturizing treatment is a treatment for depositing a water-soluble component on a nonwoven fabric.

The water-soluble component can be deposited on the nonwoven fabric by applying a moisturizing liquid to a raw nonwoven fabric (pre-moisturized nonwoven fabric). The moisturizing liquid can contain moisture-retaining components, water, oils, and surfactants for dispersing the oils in the moisturizing liquid. When the moisturizing liquid is applied in a state where the water content of the moisturizing liquid is adjusted to an amount near the equilibrium moisture regain, drying and moisture conditioning steps after application can be omitted, and accordingly, it is economical.

Generally, a moisturizing liquid is applied to a nonwoven fabric. The application method may be selected from common application methods such as gravure coating, spray coating, die coating, dipping, or the like. One-side application may be possible, however both-side application is preferable to make the skin touch in the front and rear faces of the nonwoven fabric even.

In the case the moisturizing liquid is applied while the water component ratio is kept in an equilibrium state by adding water, the drying or moisture conditioning step can be omitted after the coating step and therefore, it is economical.

At the time of application of the moisturizing liquid, it is preferable to control the temperature of the moisturizing liquid. Since viscosity of the moisturizing liquid is sometimes changed depending on the temperature, it becomes difficult to produce a product with a uniform application amount unless the temperature is controlled. Although the temperature differs depending on the blending of the moisturizing liquid, it may be set generally in a range from 10 to 60°C and preferably in a range from 30 to 50°C.

[Moisturizing liquid]

The moisturizing liquid contains a moisturizing agent, which is a moisture-retaining component, and water. If necessary, other additives may be added.

The moisture-retaining component is a component which has a high water retention capability. Practical examples of the moisturizing agent are glycerin, diglycerin, polyglycerin, ethylene glycol, diethylene glycol, polyethylene glycol, propylene glycol, 1,3-butylene glycol, sorbitol, xylitol, erythritol, mannitol, lactitol, oligosaccharide alcohol, maltitol, reduced starch hydrolysis product, fructose, glucose, oligosaccharide, trehalose, glycine betaine, pyrrolidonecarboxylic acid, pyrrolidone carboxylic acid salt, hyaluronic acid, hyaluronic acid salt, lactic acid, lactic acid salt, and urea.

Glycerin is preferable in terms of moisture absorption, cost, and safety. Although inferior to glycerin in the moisture absorption, sorbitol has a strong effect of retaining water which is once taken and when it is used in combination with glycerin, the effect of buffering change of the water content in the moisturized nonwoven fabric against the fluctuation of the ambient moisture can be obtained.

The moisturizing liquid may further contain oils.

Practical examples of oils are hydrocarbons such as liquid paraffin and squalane; fats and oils such as olive oil, camellia oil, castor oil, soybean oil, coconut oil, beef tallow, tri(caprylic acid-capric acid)glycerin, and tricaprylic acid glycerin; and ester oils such as isopropyl myristate, isopropyl palmitate, and cetyl octanoate. The oils are preferable in liquid phase at 35°C.

Addition of the oils is excellent in improvement of the touch, the moisture retention effect by sealing the skin, the buffering effect on the water fluctuation, and the buffering effect on change of the touch due to the water fluctuation.

Additives to be used for the moisturizing liquid may be fatty acids, higher alcohols, silicones, and waxes. These components improve the viscidity of the moisture-retaining component and improve the smoothness.

The fatty acids practically include fatty acids, fatty acid salts, and glycerin fatty acid esters. Examples of the fatty acids are stearic acid, palmitic acid, myristic acid, lauric acid, and caprylic acid. Examples of the fatty acid salts are sodium salts, potassium salts, triethanolamine salts, diethanolamine salts, and monoethanolamine salts of these fatty acids. Examples of the glycerin fatty acid esters are glycerin monofatty acid esters and polyglycerin fatty acid esters of these fatty acids.

Examples of the higher alcohols are lauryl alcohol, myristyl alcohol, cetanol, stearyl alcohol, octyldodecanol, and behenyl alcohol.

The silicones include modified silicone oils such as amino-modified, epoxy-modified, carboxyl-modified, polyether modified, and polyglycerin-modified oils and dimethylpolysiloxane. The waxes include bees wax, carnauba wax, and lanolin.

The water contained in the moisturized nonwoven fabric is water which is retained by the moisture absorption capability of the moisture-retaining component or the basic paper of the nonwoven fabric. That is, it is water in an equilibrium state with the water existing in ambient air in which the moisturized nonwoven fabric is placed. Therefore, the moisturized nonwoven fabric is essentially different from products such as wet tissues which contain 200% or more of water with respect to the weight of the nonwoven fabric and require a wrapping container for prevention of drying. However, the moisturized nonwoven fabric may be sealed and wrapped, or further wrapped in a state where the water content is even higher or lower in order to keep the fabric sanitary and keep good moisture feeling. The water content of the wrapped moisturized nonwoven fabric is preferably (the equilibrium moisture regain at 23°C and 50%RH + 40%) or lower, more preferably (the equilibrium moisture regain at 23°C and 50%RH + 20%) or lower, and most preferably (the equilibrium moisture regain at 23°C and 50%RH + 10%) or lower.

The moisturized nonwoven fabric of the invention may include a nonwoven fabric containing 10% or more of fibers with a length of 20 to 100 mm and a water-soluble component deposited on the nonwoven fabric.

[Fibers]

Various fiber materials used for producing nonwoven fabrics may be used alone or in combination.

Examples of fiber materials are cellulose fibers, rayon fibers, synthetic fibers, and semi-synthetic fibers.

[Cellulose fibers]

If hydrophilic cellulose fibers are used as the fibers, the touch and water retention property can be made preferable.

Natural cellulose fibers may be used as the cellulose fibers. Use of the natural cellulose fibers improves the water absorption and water retention property of the nonwoven fabric. Examples of the natural cellulose are wood pulp, non-wood pulp, cotton, cotton linter, and bast fiber. Examples of the wood pulp include softwood pulp and hardwood pulp. Examples of the non-wood pulp include pulp of bamboo, straw, and bagasse. Examples of bark fibers are kozo (paper mulberry), mitsumata, hemp, jute, and ganpi (Wikstroemia sikokiana).

Especially, softwood pulp is most preferable since it is economical, has long fiber length as compared with hardwood pulp, and is not lost at the time of a hydroentangled treatment or at the time of use.

Addition of pulp to the fiber materials composing the nonwoven fabric gives softness and moist feeling at the time of moisture retention process. Further, the water absorption speed is accelerated and the water absorption amount is increased, and at the time of wiping, the amount of water remaining in the wiped object is lessened. The addition amount of pulp may be set in 5 to 90% in the entire fibers composing the nonwoven fabric. It is preferably 20 to 80% and more preferably 40 to 70%. If the addition amount of pulp is too high, the strength of the nonwoven fabric is decreased.

Natural cellulose fibers may not be added. If the addition amount of natural cellulose fibers is 5% or lower, it becomes easy to transfer the moisturizing liquid to the skin.

[Rayon fibers]

Rayon fibers, which are regenerated cellulosic fibers, may be used. Examples of rayon fibers are cupra rayon and viscose rayon.

If rayon fibers are added to the fiber materials of the nonwoven fabric, the strength is increased, water absorption is improved, and soft touch is provided by hydroentangling. The addition amount of the rayon fibers may be 10 to 100%, preferably 20 to 80%, and more preferably 25 to 75%.

The softwood pulp and the rayon fibers may be combined. Addition of both fibers gives the nonwoven fabric with good balance among the touch, strength, and water absorption.

[Synthetic fibers]

Synthetic fibers made of various kinds of synthetic resin materials can be used.

Examples of the synthetic fibers are homopolymers and copolymers of polyesters, polyamides, polyacrylonitriles, polyolefins, and polyvinyl alcohols. More particularly, examples are polyethylene terephthalate (PET), polytetramethylene terephthalate (PTT), polypropylene, and thermofusible fibers. If, for example, PET, PTT, or polypropylene is added, the thickness feeling and soft feeling can be improved.

The addition amount of the synthetic fibers may be 0 to 50%, preferably 5 to 45%, and more preferably 5 to 30%. If the addition amount exceeds 50%, the water absorption tends to be easily decreased. However, even if the water absorption is deteriorated, in order to make it easy to transfer the moisturizing component to the human skin, the addition amount of the synthetic fibers may be increased to 100%.

To prevent depletion of fibers, thermofusible fibers may be added to carry out thermal bonding. The addition amount of the thermofusible fibers is preferably 30% or lower. If the addition amount of the thermofusible fibers is increased more than that, the sheet may become hard and the water absorption is decreased.

To improve the function of transferring the liquid contained in the nonwoven fabric, it is preferable to use oleophilic synthetic fibers. The transferring property of the moisturizing liquid to the skin is improved and moisture retention property of the skin is improved. If hydrophilic fibers such as the above-mentioned natural cellulose fibers and oleophilic fibers are used in combination, the functions of both fibers may be extracted in a good balance.

[Natural fibers]

Examples of natural fibers other than the above-mentioned natural cellulose fibers may be wool and silk. Examples of the semi-synthetic fibers may be cellulose acetate fibers. Composite fibers obtained by compounding a plurality of fiber materials may also be usable. A single fiber layer may be composed by combining a plurality of fiber materials.

[Other fibers]

Segmental fibers easy to be divided into segments such as Wramp fibers (manufactured by Kuraray Co., Ltd.) may be added. Addition of the segmental fibers increases the strength and good surface property. Since the water pressure cannot be increased in the hydroentangling treatment to produce a soft sheet, segmental fibers easy to be divided at around 5 MPa are preferable to be added.

[Fineness]

The fineness of the fiber materials with a fiber length of 20 to 100 mm may be 0.5 to 10.0 dtex, preferably 0.7 to 5.0 dtex, and more preferably 0.8 to 2.5 dtex.

The nonwoven fabric produced from fibers with low fineness gives soft and smooth touch and is therefore preferable. However, staple fibers with fineness of 0.7 dtex or lower cannot be processed by a carding machine to make the production of fiber webs difficult and thus the staple fibers are hardly usable. In the case of segmental fibers, there is no problem that the fineness becomes low after segmentation if the fineness condition is satisfied before segmentation.

If fibers with high fineness are used, the nonwoven fabric excellent in elastic feeling and thickness feeling can be produced. However, fibers with so high fineness give pricking feeling on the skin or tend to cause inflammation by physical stimulation.

It is preferable to add 20 to 80% by weight of fibers with a fineness of 0.5 to 10.0 dtex in the entire fiber materials composing the nonwoven fabric.

[Length of fibers]

The nonwoven fabric contains 10% or more of fibers with a length of 20 to 100 mm to the entire fiber materials composing the nonwoven fabric. The amount of the fibers with a length of 20 to 100 mm is preferably 20% or more to obtain a nonwoven fabric excellent in the drape property (property of shaping along the skin).

In production of the nonwoven fabric, in the case of producing webs in a drylaid method, it is preferable to set the length of the fiber materials in a range from 20 to 100 mm. The length of the fiber materials is more preferably 35 to 60 mm. If the length is shorter or longer than the above-mentioned range, it becomes hard to process such fibers with a carding machine. In the case of producing a fiber layer by a wetlaid method, it is preferable to use staple fibers with a fiber length shorter than 20 mm.

The nonwoven fabric may contain 20 to 80% by weight of fibers with a fiber length of 20 to 100 mm and fineness of 0.5 to 10.0 dtex and 20 to 80% by weight of natural cellulose fibers with a fiber length shorter than 20 mm. It is more preferable that the nonwoven fabric may contain 20 to 50% by weight of fibers with a fiber length of 20 to 100 mm and fineness of 0.5 to 10.0 dtex and 50 to 80% by weight of natural cellulose fibers with a fiber length shorter than 20 mm.

[Pulp paper]

In the case the above-mentioned fiber materials such as cellulose fibers hard to be processed by a carding machine to produce fiber webs and accordingly produce the nonwoven fabric are used, pulp paper may be produced.

Pulp paper can be produced by dispersing natural cellulose fibers or the like in water and making a sheet from the fibers by a conventional method. Pulp paper made to have a sheet form by an airlaid method is also usable.

Pulp paper may contain other fiber materials such as thermofusible fibers and synthetic pulp. Thermal bonding after compounding the fiber materials with fiber webs improves the strength of the nonwoven fabric and prevent depletion of fibers.

[Production of nonwoven fabric]

For the production of the nonwoven fabric before the water-soluble component is deposited, that is, the raw (pre-moisturized) nonwoven fabric, can be carried out using common nonwoven fabric manufacturing techniques.

Specifically, a spunlace method, a spunbonded method, a wetlaid method, an airlaid method, a chemical bonded method, and a meltblown method can be employed. These methods can also be used in combination. The spunlace method, in which the fibers are loosely bonded to each other by entangling the fibers with water current, is a preferable manufacturing technique. Inter alia, spunlace nonwoven fabrics containing cellulose fibers such as pulp and rayon are preferable because they have good water absorption property and can give increased moisture feeling.

METSUKE of the raw nonwoven fabric to be subjected to a moisturizing treatment may be set at 20 to 100 $g/m^2$. In the case of wiping delicate parts, such as the surroundings of eyes, for which the softness and drape property are thought to be important, METSUKE is preferably in a range from 30 to 60 $g/m^2$ and more preferably in a range from 40 to 50 $g/m^2$. On the other hand, in the case of wiping the entire bodies for which high water absorption amount and strength are thought important, it is preferably in a range from 40 to 100 $g/m^2$ and more preferably in a range from 50 to 80 $g/m^2$.

When blending pulp in the fiber composing the nonwoven fabric, fiber webs are produced by a carding machine using fibers with a fiber length of 20 mm or longer. Then, the separately produced pulp paper may be superimposed to produce nonwoven fabric by compounding using a hydroentangling method.

Although the wetlaid method and the airlaid method may also be employed, fibers with a fiber length of 20 mm or longer are hard to be employed for these methods and thermal bonding or chemical bonding is required to give strength. Consequently, the resulting nonwoven fabric may be hard and have problems of inferior drape property, stiffness, lack of elongation, and easiness of tear in use.

The spun bonding method and the melt blow method may also be employed. The nonwoven fabric produced by these methods is excellent in the strength, however generally it gives hard feeling and is usually poor in the drape property.

In the case of producing the nonwoven fabric by compounding the fiber webs and pulp paper, the nonwoven fabric is produced generally by compounding in a three-layer structure of web/pulp paper/web. Accordingly, the following problems can be avoided at the time of the hydroentangling treatment: that is, the pulp paper is stuck to a conveyer belt and hard to be peeled off the belt and that pulp is lost by the water current for the hydroentangling to worsen the surface property.

The nonwoven fabric may be produced in a two-layer structure of web/pulp paper. That is advantageous in the case of producing a sheet with a large addition amount of pulp.

The nonwoven fabric may be produced in a four-layer structure of web/pulp paper/pulp paper/web or may be produced in a larger numbers of layer structures.

The nonwoven fabric can be produced without compounding pulp by producing fiber webs by a carding machine from fibers with a fiber length of 20 mm or longer and thereafter producing the nonwoven fabric by the hydroentangling method.

[Hydroentangling treatment]

As a production technique of the spunlace nonwoven fabric, the hydroentangling treatment employed for drylaid or wetlaid production or layer formation of the nonwoven fabric can be employed.

The hydroentangling treatment is a treatment carried out to entangling fibers composing the webs and densifying the entangling by jetting high pressure water current in a column state to the fiber webs supported on a water-permeable mesh-type support net through the fine holes and penetrating the fiber webs with the water current.

As the support net is generally used an endlessly turning mesh-type conveyer belt and while the webs are conveyed by the conveyer belt, the entangling and uniting treatment is carried out with the water current.

If the hydroentangling treatment is carried out by penetrating the layered fiber webs and pulp paper with the water current, the fiber webs and the pulp paper can be entangled and united. The hydroentangling treatment entangles the fibers composing the webs and fibers composing the pulp paper in the state that both fibers mutually penetrate the layers of the counterparts and produces the nonwoven fabric in which fibers are practically united.

The treatment conditions of the hydroentangling treatment differ depending on the materials and structures of the fiber webs and pulp paper, METSUKE, and thickness.

[Conveyer belt]

The conveyer belt to be employed in the hydroentangling treatment is a mesh-type conveyer belt with an 8 to 125 mesh number. Mesh materials such as metals and synthetic fibers may be used for the mesh-type conveyer belt.

The entangling state of the fibers of the fiber webs and the pulp paper differ according to the size of the water flow space of the mesh-type conveyor belt. In addition, the structure of the treated nonwoven fabric depends on the form of the water current passing through the water flow space.

If the mesh-type conveyer belt has the mesh number in the above-mentioned range, the fiber webs and the pulp paper can efficiently be entangled and united. If the mesh number is too large (that is, the meshes become small), since the entangling and uniting the fiber web and the pulp paper cannot sufficiently be carried out, the strength of the nonwoven fabric to be obtained is decreased. If the mesh number is too small (that is, the meshes become rough), the fibers of the pulp paper are depleted much and therefore, it is not preferable. Further in the case of using the nonwoven fabric as a wiper (a wiping sheet), stains easily pass through the meshes and come out. The mesh number is preferably in a range from 20 to 100. If it is about 20 meshes, the surface of the nonwoven fabric is provided with proper gauze-like unevenness and in the case of using the nonwoven fabric as a wiper, the stains are easy to be caught. If the mesh number is about 100 meshes, the nonwoven fabric with smooth touch can be obtained. The mesh number may be controlled in accordance with the uses.

[Nozzle for hydroentangling]

The diameter of orifices or holes of a nozzle for jetting water current can be set to be 0.05 to 0.30 mm and preferably 0.07 to 0.16 mm. If the diameter is smaller, the nonwoven fabric is provided with a fine surface quality. However, if the diameter is too small, it becomes difficult to sufficiently carry out entangling of fibers. If the diameter is too large, the nonwoven fabric may have holes or the surface quality may be deteriorated.

The intervals of the holes can be set in a range from 0.5 to 2 mm. As the intervals are narrow, the entangling of the fibers by water current can be carried out densely, however the arrangement of the holes becomes difficult. If the intervals becomes too wide, portions where entangling is insufficiently carried out may be increased between neighboring holes. The holes may be arranged at the above-mentioned intervals in a single row or in a plurality of rows.

A nozzle with 2 to 20 mm intervals may be used for making stains easy to be caught in the case of using the nonwoven fabric as a wiper.

[Water current]

Generally water is used for water current, but hot water and steam can also be used.

The pressure of the water current may be set to 0.5 to 10 Mpa. If the pressure is larger, the function of entangling the fibers is strengthened, however, if the pressure is too large, the nonwoven fabric may have holes or the pulp may be lost.

The nonwoven fabric may be produced by only one time hydroentangling treatment or by repeating the hydroentangling treatment a plurality of times under the same treatment conditions or under changed treatment conditions with different treatment apparatus. To prevent fiber depletion and fluffing, it is preferable to carry out the hydroentangling treatment at least one time from both sides.

[Drying]

After the hydroentangling treatment, a dewatering process and a drying process may be carried out by a common method. The drying temperature is preferably 40 to 200°C. If the temperature is too low, the production cannot be carried out economically at a proper speed. If the temperature is too high, rayon fibers or pulp may possibly be burned.

In the case thermofusible fibers are added, the thermofusible fibers are melted to carry out thermal bonding. It may be carried out by an air-through drier, a Yankee drier, or hot press rolls. Even if no thermofusible fiber is added, the nonwoven fabric may be hot-pressed by being passed though hot press rolls to make the surface smooth and suppress fluffing.

[Embossing]

The moisturized nonwoven fabric may be embossed.

Common embossing techniques for nonwoven fabrics may be employed in this case.

[Application of moisturized nonwoven fabric]

The moisturized nonwoven fabric can be used in uses similar to conventional tissue paper, paper towels, moisturized tissues, and wet tissues.

Practically the moisturized nonwoven fabric may be used as wiping sheets for wiping the backside of a baby and wiping mouth and hands of a baby; cleaning sheets for the elderly nursing; cleaning sheets for wiping mouth of the elderly people; cosmetic paper for anti-shining; and facial care sheets for females.

The moisturized nonwoven fabric of the invention is excellent in the skin touch and wiping property as well as the functionalities of water absorption property and water retention property. The moisturized nonwoven fabric of the invention is also excellent in the functionalities of moisturizing liquid transfer property and skin moisturizing effect.

With respect to the moisturized nonwoven fabric of the invention, since a water-soluble component is deposited on a nonwoven fabric containing a sufficient amount of fibers with a relatively long fiber length, which are not used in the common moisturized tissues, the moisturized nonwoven fabric of the invention can solve the problems which conventional moisturized tissues and wet tissues have.

More practically, the moisturized nonwoven fabric has sufficient strength despite its softness and a pleasant texture. The moisturized nonwoven fabric is capable of keeping a proper amount of water and efficiently wiping out cosmetics or the like without giving unpleasant cooling or irritating feel to the skin. The moisturized nonwoven fabric is also capable of continuously keeping the moisture retention property for a long duration without being dried even if it is left in an opened state.

As a result, the moisturized nonwoven fabric can be used for various purposes for which conventional moisturized tissues or wet tissues cannot be used. The moisturized nonwoven fabric can be used preferably in a wide range of uses, for example, as wiping fabrics for nursing care, cosmetic webs, moisturizing sheets, and wiping of articles and is thus provided with high value in practical use.

DETAILED DESCRIPTION OF THE INVENTION

Hereinafter, the moisturized nonwoven fabric of the invention will be described in detail along with Examples.

[Embodiment 1]

(Example 1)

Softwood kraft pulp (Howe Sound 400, manufactured by Canfor Corporation) was disintegrated by a pulper to produce pulp paper with about 30 g/m² by a cylinder type paper manufacture machine. Rayon fibers (fineness 1.1 dtex $\times$ fiber length 38 mm) were disintegrated by two carding machines to produce a pair of fiber webs with about 10 g/m² METSUKE. The above-mentioned pulp paper was sandwiched between the pair of fiber webs and fed to a conveyer belt. The pulp paper and the webs were subjected to entangling and uniting treatment by a water jet treatment apparatus installed on the path of the conveyer belt to obtain pulp-containing spunlace nonwoven fabric with about 52.4 g/m² METSUKE. The water jet treatment was carried out using a nozzle having holes with a diameter of 80 $\mu$m at 1 mm

pitches (a water jetting apparatus having small holes with a diameter of 0.08 mm and arranged in a row at 1 mm intervals) at water pressure of 4.5 MPa.

Then, the nonwoven fabric produced in the above-mentioned manner was moisturized. A moisturizing liquid was applied to the nonwoven fabric by a spray coating method. Three types of moisturized nonwoven fabrics were produced with having different amounts of the moisturizing liquid. After air dried and moisture conditioning, the obtained moisturized nonwoven fabrics were subjected to measurement to find that METSUKE was 68.4 (Example 1-1), 74.4 (Example 1-2), and 107.5 g/m$^2$ (Example 1-3).

The moisturizing liquid was produced by mixing 60% by weight of glycerin, 10% by weight of sorbitol, 1% by weight of decaglycerin monostearic acid ester, 5% by weight of liquid paraffin, 1% by weight of polyoxyethylene (20 EO) sorbitan monostearate, 0.4% by weight of sorbitan monostearate, and 22.6% by weight of water.

The nonwoven fabric before the moisturizing treatment (the pre-moisturized nonwoven fabric) was employed as Comparative Example 1.

Table 1 shows the results of property evaluation tests and sensory tests of Examples 1-1, 1-2, 1-3, and Comparative Example 1. The property evaluation tests and the sensory tests for Examples and Comparative Example were carried out as follows.

[Table 1]

| | | Example 1-1 | Example 1-2 | Example 1-3 | Comparative Example 1 |
|---|---|---|---|---|---|
| METSUKE | g/m$^2$ | 68.4 | 74.4 | 107.5 | 52.4 |
| content of water-soluble component | % | 30.4 | 41.9 | 105.0 | 0.0 |
| equiliblium water content | % | 10.5 | 11.1 | 13.2 | 8.1 |
| increase ratio of equiliblium water content | % | 2.4 | 3.0 | 5.1 | 0.0 |
| strength (vertical) | N | 16.5 | 21.4 | 24.9 | 40.1 |
| (transverse) | N | 7.8 | 8.9 | 11.1 | 15.4 |
| elongation (vertical) | % | 29.1 | 25.8 | 29.4 | 18.1 |
| (transverse) | % | 77.4 | 82.4 | 79.8 | 68.5 |
| F5 (vertical) | N | 5.4 | 6.4 | 5.9 | 26.6 |
| (transverse) | N | 0.59 | 0.61 | 0.57 | 2.14 |
| F5/METSUKE (vertical) | N·m$^2$/g | 0.078 | 0.086 | 0.055 | 0.506 |
| Larose method | | | | | |
| 5 second-water absorption/g | ml/g | 0.28 | 0.19 | 0.06 | 6.31 |
| 10 second-water absorption/g | ml/g | 0.40 | 0.31 | 0.12 | 6.90 |
| maximum water absorption/g | ml/g | 1.65 | 1.55 | 1.12 | 7.15 |
| water retention ratio | - | 6.39 | 6.07 | 4.29 | 9.83 |
| cantilever method | | | | | |
| bending resistance/ METSUKE (vertical) | mm·m$^2$/g | 1.11 | 0.81 | 0.54 | 2.34 |
| bending resistance/ METSUKE (transverse) | mm·m$^2$/g | 0.37 | 0.36 | 0.24 | 0.77 |

(continued)

| | | Example 1-1 | Example 1-2 | Example 1-3 | Comparative Example 1 |
|---|---|---|---|---|---|
| handle O-meter method | | | | | |
| bending resistance/ METSUKE (vertical) | $mN \cdot m^2/g$ | 1.96 | 2.14 | 1.41 | 11.18 |
| bending resistance/ METSUKE (transverse) | $mN \cdot m^2/g$ | 0.45 | 0.51 | 0.48 | 1.54 |
| drape coefficient | % | 62 | 58 | 61 | 76 |
| KES surface MIU | - | 0.347 | 0.350 | 0.189 | 0.521 |
| MMD | - | 0.010 | 0.010 | 0.008 | 0.015 |
| KES bending B (vertical) | $gf \cdot cm^2/cm$ | 0.1026 | 0.0884 | 0.0853 | 0.5140 |
| B (transverse) | $gf \cdot cm^2/cm$ | 0.0136 | 0.0139 | 0.0196 | 0.0431 |
| KES bending 2HB (vertical) | $gf \cdot cm/cm$ | 0.1422 | 0.1303 | 0.1616 | 0.5173 |
| 2HB (transverse) | gf cm/cm | 0.0137 | 0.0153 | 0.0287 | 0.0233 |
| KES shear G (vertical) | $gf/cm \cdot degree$ | 1.26 | 1.28 | 1.22 | 3.27 |
| G (transverse) | $gf/cm \cdot degree$ | 1.26 | 1.15 | 1.25 | 3.36 |
| KES shear 2HG (vertical) | gf/cm | 2.20 | 2.84 | 4.09 | 4.68 |
| 2HG (transverse) | gf/cm | 4.62 | 5.06 | 5.66 | 6.09 |
| Qmax | $J/cm^2/sec$ | 0.112 | 0.115 | 0.128 | 0.076 |
| sensory tests | | | | | |
| softness | | good | good | Very good | poor |
| smoothness | | good | Very good | Very good | poor |
| moisture feeling | | good | Very good | Very good | poor |
| skin moisturizing property | | good | good | Very good | poor |
| wiping property | | good | good | good | average |

(Example 2)

Softwood kraft pulp (Howe Sound 400, manufactured by Canfor Corporation) was disintegrated by a pulper to produce pulp paper with about 30 g/m² by a cylinder type paper manufacture machine. Mixed fibers containing 50% by weight of rayon fibers (fineness 1.1 dtex × fiber length 38 mm) and 50% by weight of PET fibers (fineness 1.1 dtex × fiber length 38 mm) were disintegrated by two carding machines to produce a pair of fiber webs with about 10 g/m² METSUKE. The above-mentioned pulp paper was sandwiched between the pair of fiber webs and fed to a conveyer belt. The pulp paper and the webs were subjected to entangling and uniting treatment by a water jet treatment apparatus installed on the path of the conveyer belt to obtain pulp-containing spunlace nonwoven fabric with about 50.2 g/m² METSUKE. The water jet treatment was carried out using a nozzle having holes with a diameter of 80 μm at 1 mm pitches and water pressure of 4.5 MPa.

Then, the nonwoven fabric produced in the above-mentioned manner was moisturized. The moisturizing liquid was applied to the nonwoven fabric by a spray coating method. Three types of treated nonwoven fabrics were produced by changing the application amounts of the moisturizing liquid. After air dried and moisture conditioning, the obtained moisturized nonwoven fabrics were subjected to measurement to find that METSUKE was 58.2 (Example 2-1), 71.6 (Example 2-2), and 103.7 g/m$^2$ (Example 2-3). The moisturizing liquid was same as that used in Example 1.

The nonwoven fabric before the moisturizing treatment (the pre-moisturized nonwoven fabric) was employed as Comparative Example 2.

Table 2 shows the results of property evaluation tests and sensory tests of Examples 2-1, 2-2, 3-3, and Comparative Example 2. The property evaluation tests and the sensory tests for Examples and Comparative Example were carried out as follows.

[Table 2]

| | | Example 2-1 | Example 2-2 | Example 2-3 | Comparative Example 2 |
|---|---|---|---|---|---|
| METSUKE | g/m$^2$ | 58.2 | 71.6 | 103.7 | 50.2 |
| content of water-soluble component | % | 15.9 | 42.7 | 106.6 | 0.0 |
| equiliblium water content | % | 8.2 | 10.1 | 12.3 | 6.0 |
| increase ratio of equiliblium water content | % | 2.2 | 4.1 | 6.2 | 0.0 |
| strength (vertical) | N | 14.9 | 22.1 | 23.1 | 46.0 |
| (transverse) | N | 3.1 | 5.1 | 5.8 | 11.9 |
| elongation (vertical) | % | 29.5 | 28.3 | 32.5 | 27.6 |
| (transverse) | % | 53.9 | 87.8 | 93.9 | 81.8 |
| F5 (vertical) | N | 7.2 | 6.8 | 5.5 | 23.2 |
| (transverse) | N | 0.56 | 0.41 | 0.36 | 1.23 |
| F5/METSUKE (vertical) | N·m$^2$/g | 0.124 | 0.095 | 0.053 | 0.463 |
| Larose method | | | | | |
| 5 second-water absorption/g | ml/g | 1.14 | 0.33 | 0.13 | 5.29 |
| 10 second-water absorption/g | ml/g | 1.59 | 0.49 | 0.23 | 6.59 |
| maximum water absorption/g | ml/g | 2.89 | 1.92 | 1.46 | 7.34 |
| water retention ratio | - | 7.94 | 6.04 | 4.48 | 9.45 |
| cantilever method | | | | | |
| bending resistance/METSUKE (vertical) | mm·m$^2$/g | 1.32 | 0.92 | 0.51 | 1.86 |
| bending resistance/METSUKE (transverse) | mm·m$^2$/g | 0.43 | 0.32 | 0.21 | 0.65 |
| handle O-meter method | | | | | |
| bending resistance/METSUKE (vertical) | mN·m$^2$/g | 3.46 | 1.90 | 1.31 | 9.42 |
| bending resistance/METSUKE (transverse) | mN·m$^2$/g | 0.58 | 0.42 | 0.39 | 1.00 |
| drape coefficient | % | 53 | 51 | 51 | 70 |
| KES surface MIU | - | 0.444 | 0.378 | 0.186 | 0.480 |
| MMD | - | 0.008 | 0.011 | 0.008 | 0.014 |
| KES bending     B (vertical) | gf·cm$^2$/cm | 0.1460 | 0.0969 | 0.0739 | 0.3283 |
| (transverse)     B | gf·cm$^2$/cm | 0.0163 | 0.0148 | 0.0143 | 0.0291 |

(continued)

| | | | Example 2-1 | Example 2-2 | Example 2-3 | Comparative Example 2 |
|---|---|---|---|---|---|---|
| KES bending (vertical) | 2HB | gf·cm/cm | 0.2131 | 0.1379 | 0.1368 | 0.4056 |
| (transverse) | 2HB | gf·cm/cm | 0.0140 | 0.0154 | 0.0195 | 0.0194 |
| KES shear (vertical) | G | gf/cm·degree | 1.53 | 1.16 | 1.05 | 2.81 |
| (transverse) | G | gf/cm·degree | 1.38 | 1.08 | 0.96 | 3.09 |
| KES shear (vertical) | 2HG | gf/cm | 2.30 | 2.52 | 3.09 | 3.82 |
| (transverse) | 2HG | gf/cm | 4.87 | 4.66 | 4.59 | 6.14 |
| Qmax | | $J/cm^2/sec$ | 0.093 | 0.108 | 0.117 | 0.071 |
| sensory tests | | | | | | |
| softness | | | good | Very good | Very good | poor |
| smoothness | | | Very good | Very good | Very good | poor |
| moisture feeling | | | good | Very good | Very good | poor |
| skin moisturizing property | | | average | good | Very good | poor |
| wiping property | | | good | good | good | average |

(Example 3)

Softwood kraft pulp (Howe Sound 400, manufactured by Canfor Corporation) was disintegrated by a pulper to produce pulp paper with about 25 $g/m^2$ by a cylinder type paper manufacture machine. Rayon fibers (fineness 1.7 dtex × fiber length 40 mm) were disintegrated by two carding machines to produce a pair of fiber webs with about 13 $g/m^2$ METSUKE. The above-mentioned pulp paper was sandwiched between the pair of fiber webs and fed to a conveyer belt. The pulp paper and the webs were subjected to entangling and uniting treatment by a water jet treatment apparatus installed on the path of the conveyer belt to obtain pulp-containing spunlaced nonwoven fabric with about 51.7 $g/m^2$ METSUKE. The water jet treatment was carried out using a nozzle having holes with a diameter of 100 μm at 1 mm pitches and water pressure of 5.0 MPa.

Then, the nonwoven fabric produced in the above-mentioned manner was moisturized. The moisturizing liquid was applied to the nonwoven fabric by a gravure coating method. After air dried and moisture conditioning, the obtained moisturized nonwoven fabric was subjected to measurement to find that METSUKE was 70.1 $g/m^2$. The moisturizing liquid was same as that used in Example 1.

The nonwoven fabric before the moisturizing treatment (the pre-moisturized nonwoven fabric) was employed as Comparative Example 3.

Table 3 shows the results of property evaluation tests and sensory tests of Example 3 and Comparative Example 3. The property evaluation tests and the sensory tests for Example and Comparative Example were carried out as follows.

(Example 4)

Softwood kraft pulp (Howe Sound 400, manufactured by Canfor Corporation) was disintegrated by a pulper to produce pulp paper with about 32 $g/m^2$ by a cylinder type paper manufacture machine. Mixed fibers containing 50% by weight of rayon fibers (fineness 1.7 dtex × fiber length 40 mm) and 50% by weight of PET fibers (fineness 1.6 dtex × fiber length 44 mm) were disintegrated by one carding machine to produce fiber webs with about 14 $g/m^2$ METSUKE. The above-mentioned pulp paper was laid on the fiber webs and fed to a conveyer belt. The pulp paper and the webs were subjected to entangling and uniting treatment by a water jet treatment apparatus installed on the path of the conveyer belt to obtain pulp-containing spunlace nonwoven fabric with about 45.5 $g/m^2$ METSUKE. The water jet treatment was carried out using a nozzle having holes with a diameter of 100 μm at 1 mm pitches and water pressure of 3.5 MPa.

Then, the nonwoven fabric produced in the above-mentioned manner was moisturized. The moisturizing liquid was applied to the nonwoven fabric by a gravure coating method. After air dried and moisture conditioning, the obtained moisturized nonwoven fabric was subjected to measurement to find that METSUKE was 57.0 $g/m^2$. The moisturizing liquid was same as that used in Example 1.

The nonwoven fabric before the moisturizing treatment (the pre-moisturized nonwoven fabric) was employed as Comparative Example 4.

Table 3 shows the results of property evaluation tests and sensory tests of Example 4 and Comparative Example 4. The property evaluation tests and the sensory tests for Example and Comparative Example were carried out as follows.

[Table 3]

| | | Example 3 | Comparative Example 3 | Example 4 | Comparative Example 4 |
|---|---|---|---|---|---|
| METSUKE | $g/m^2$ | 70.1 | 51.7 | 57.0 | 45.5 |
| content of water-soluble component | % | 35.7 | 0.0 | 25.2 | 0.0 |
| equiliblium water content | % | 10.6 | 7.8 | 9.0 | 5.9 |
| increase ratio of equiliblium water content | % | 2.8 | 0.0 | 3.1 | 0.0 |
| strength (vertical) | N | 18.2 | 34.9 | 14.3 | 43.4 |
| (transverse) | N | 9.8 | 19.4 | 1.7 | 7.8 |
| elongation (vertical) | % | 32.1 | 21.0 | 22.1 | 23.7 |
| (transverse) | % | 69.2 | 60.7 | 50.0 | 65.6 |
| F5 (vertical) | N | 5.1 | 21.9 | 6.9 | 23.4 |
| (transverse) | N | 0.55 | 2.55 | 0.38 | 1.48 |
| F5/METSUKE (vertical) | $N \cdot m^2/g$ | 0.073 | 0.423 | 0.121 | 0.514 |
| Larose method | | | | | |
| 5 second-water absorption/g | ml/g | 0.25 | 5.30 | 1.25 | 3.19 |
| 10 second-water absorption/g | ml/g | 0.41 | 6.69 | 1.82 | 4.05 |
| maximum water absorption/g | ml/g | 1.77 | 6.91 | 3.72 | 6.48 |
| water retention ratio | - | 6.12 | 9.29 | 6.67 | 8.97 |
| cantilever method | | | | | |
| bending resistance/METSUKE (vertical) | $mm \cdot m^2/g$ | 0.62 | 1.71 | 0.86 | 1.93 |
| bending resistance/METSUKE (transverse) | $mm \cdot m^2/g$ | 0.32 | 0.62 | 0.38 | 0.62 |
| handle O-meter method | | | | | |
| bending resistance/METSUKE (vertical) | $mN \cdot m^2/g$ | 1.11 | 7.44 | 1.76 | 6.34 |
| bending resistance/METSUKE (transverse) | $mN \cdot m^2/g$ | 0.39 | 1.07 | 0.39 | 0.73 |
| drape coefficient | % | 45 | 72 | 43 | 68 |
| KES surface MIU | - | 0.425 | 0.626 | 0.377 | 0.631 |
| MMD | - | 0.011 | 0.018 | 0.011 | 0.021 |
| KES bending B (vertical) | $gf \cdot cm^2/cm$ | 0.0567 | 0.1960 | 0.0666 | 0.2537 |
| (transverse) B | $gf \cdot cm^2/cm$ | 0.0157 | 0.0172 | 0.0082 | 0.0203 |

(continued)

| | | | Example 3 | Comparative Example 3 | Example 4 | Comparative Example 4 |
|---|---|---|---|---|---|---|
| KES bending (vertical) | 2HB | gf·cm/cm | 0.0771 | 0.1999 | 0.0977 | 0.3096 |
| (transverse) | 2HB | gf·cm/cm | 0.0177 | 0.0149 | 0.0077 | 0.0150 |
| KES shear G (vertical) | | gf/cm·degree | 1.35 | 2.31 | 1.05 | 1.32 |
| (transverse) | G | gf/cm·degree | 1.26 | 2.70 | 1.09 | 1.31 |
| KES shear (vertical) | 2HG | gf/cm | 2.84 | 4.78 | 2.95 | 3.69 |
| (transverse) | 2HG | gf/cm | 4.82 | 7.58 | 5.30 | 5.69 |
| Qmax | | $J/cm^2/sec$ | 0.103 | 0.087 | 0.127 | 0.079 |
| sensory tests | | | | | | |
| softness | | | good | poor | Very good | poor |
| smoothness | | | good | poor | good | poor |
| moisture feeling | | | good | poor | good | poor |
| skin moisturizing property | | | good | poor | good | poor |
| wiping property | | | good | average | good | average |

(Example 5)*

Rayon fibers (fineness 1.7 dtex × fiber length 40 mm) were disintegrated by two carding machines and fed to a conveyer belt. The fibers were subjected to entangling and uniting treatment by a water jet treatment apparatus installed on the path of the conveyer belt to obtain a spunlace nonwoven fabric with 46.0 $g/m^2$ METSUKE. The water jet treatment was carried out using a nozzle having holes with a diameter of 100 $\mu$m at 1 mm pitches and water pressure of 5.0 MPa.
Then, the nonwoven fabric produced in the above-mentioned manner was moisturized. The moisturizing liquid was applied to the nonwoven fabric by a gravure coating method. After air dried and moisture conditioning, the obtained moisturized nonwoven fabric was subjected to measurement to find that METSUKE was 64.4 $g/m^2$. The moisturizing liquid was same as that used in Example 1.
The nonwoven fabric before the moisturizing treatment (the pre-moisturized nonwoven fabric) was employed as Comparative Example 5.
Table 4 shows the results of property evaluation tests and sensory tests of Example 5 and Comparative Example 5. The property evaluation tests and the sensory tests for Example and Comparative Example were carried out as follows.

(Example 6)*

A raw material mixture containing 50% by weight of softwood kraft pulp (Howe Sound 400, manufactured by Canfor Corporation) and 50% by weight of rayon fibers (fineness 1.7 dtex × fiber length 10 mm) was used to produce pulp paper by a short-net paper manufacture machine. The pulp paper was fed to a conveyer belt and subjected to entangling and uniting treatment by a water jet treatment apparatus installed on the path of the conveyer belt to obtain pulp-containing spunlace nonwoven fabric with 43.2 $g/m^2$ METSUKE. The water jet treatment was carried out using a nozzle having holes with a diameter of 100 $\mu$m at 1 mm pitches and water pressure of 4 MPa.
Then, the nonwoven fabric produced in the above-mentioned manner was moisturized. The moisturizing liquid was applied to the nonwoven fabric by a gravure coating method. After air dried moisture conditioning, the obtained moisturized nonwoven fabric was subjected to measurement to find that METSUKE was 56.4 $g/m^2$. The moisturizing liquid was same as that used in Example 1.
The nonwoven fabric before the moisturizing treatment (the pre-moisturized nonwoven fabric) was employed as Comparative Example 6.
Table 4 shows the results of property evaluation tests and sensory tests of Example 6 and Comparative Example 6. The property evaluation tests and the sensory tests for Example and Comparative Example were carried out as follows.

[Table 4]

| | | Example 5 | Comparative Example 5 | Example 6 | Comparative Example 6 |
|---|---|---|---|---|---|
| METSUKE | g/m$^2$ | 64.4 | 46.0 | 56.4 | 43.2 |
| content of water-soluble component | % | 40.0 | 0.0 | 30.6 | 0.0 |
| equiliblium water content | % | 11.7 | 9.8 | 10.2 | 8.2 |
| increase ratio of equiliblium water content | % | 1.9 | 0.0 | 2.0 | 0.0 |
| strength (vertical) | N | 40.0 | 58.5 | 6.2 | 32.5 |
| (transverse) | N | 18.2 | 24.1 | 2.5 | 15.7 |
| elongation (vertical) | % | 35.9 | 26.4 | 11.6 | 22.5 |
| (transverse) | % | 72.5 | 68.1 | 22.5 | 49.1 |
| F5 (vertical) | N | 2.6 | 8.2 | 4.3 | 19.4 |
| (transverse) | N | 0.50 | 0.53 | 0.79 | 3.50 |
| F5/METSUKE (vertical) | N·m$^2$/g | 0.041 | 0.177 | 0.077 | 0.449 |
| Larose method | | | | | |
| 5 second-water absorption/g | ml/g | 0.50 | 4.15 | 1.21 | 5.77 |
| 10 second-water absorption/g | ml/g | 0.78 | 5.67 | 1.62 | 6.45 |
| maximum water absorption/g | ml/g | 2.06 | 7.02 | 3.32 | 6.64 |
| water retention ratio | - | 8.53 | 12.26 | 6.35 | 9.01 |
| cantilever method | | | | | |
| bending resistance/ METSUKE (vertical) | mm·m$^2$/g | 0.55 | 0.80 | 0.78 | 1.64 |
| bending resistance/ METSUKE (transverse) | mm·m$^2$/g | 0.41 | 0.55 | 0.39 | 0.80 |
| handle O-meter method | | | | | |
| bending resistance/ METSUKE (vertical) | mN·m$^2$/g | 1.10 | 1.44 | 1.58 | 5.11 |
| bending resistance/ METSUKE (transverse) | mN·m$^2$/g | 0.56 | 0.70 | 0.49 | 1.02 |
| drape coefficient | % | 45 | 48 | 54 | 74 |
| KES surface MIU | - | 0.214 | 0.454 | 0.281 | 0.414 |
| MMD | - | 0.007 | 0.010 | 0.010 | 0.011 |

(continued)

| | | Example 5 | Comparative Example 5 | Example 6 | Comparative Example 6 |
|---|---|---|---|---|---|
| KES bending B (vertical) | gf·cm$^2$/cm | 0.0404 | 0.0412 | 0.0656 | 0.2228 |
| B (transverse) | gf·cm$^2$/cm | 0.0135 | 0.0130 | 0.0120 | 0.0261 |
| KES bending 2HB (vertical) | gf·cm/cm | 0.0464 | 0.0337 | 0.0736 | 0.1902 |
| 2HB (transverse) | gf·cm/cm | 0.0175 | 0.0136 | 0.0125 | 0.0163 |
| KES shear G (vertical) | gf/cm·degree | 0.81 | 1.14 | 1.23 | 3.09 |
| G (transverse) | gf/cm·degree | 0.87 | 0.95 | 1.49 | 3.33 |
| KES shear 2HG (vertical) | gf/cm | 2.54 | 2.46 | 3.24 | 4.88 |
| 2HG (transverse) | gf/cm | 3.98 | 3.69 | 4.93 | 7.55 |
| Qmax | J/cm$^2$/sec | 0.090 | 0.071 | 0.105 | 0.072 |
| sensory tests | | | | | |
| softness | | Very good | good | good | poor |
| smoothness | | Very good | average | good | average |
| moisture feeling | | average | poor | good | poor |
| skin moisturizing property | | good | poor | good | poor |
| wiping property | | good | average | good | average |

(Example 7)*

A raw material mixture containing 50% by weight of softwood kraft pulp (Howe Sound 400, manufactured by Canfor Corporation) and 50% by weight of rayon fibers (fineness 1.7 dtex $\times$ fiber length 10 mm) was used to produce pulp paper by a short-net paper manufacture machine. The pulp paper was fed to a conveyer belt and subjected to entangling and uniting treatment by a water jet treatment apparatus installed on the path of the conveyer belt to obtain pulp-containing spunlace nonwoven fabric with 51.1 g/m$^2$ METSUKE. The water jet treatment was carried out using a nozzle having holes with a diameter of 100 $\mu$m at 1 mm pitches and water pressure of 4 MPa.

Then, the nonwoven fabric produced in the above-mentioned manner was moisturized. The moisturizing liquid was applied to the nonwoven fabric by a gravure coating method. After air dried moisture conditioning, the obtained moisturized nonwoven fabric was subjected to measurement to find that METSUKE was 74.3 g/m$^2$. The moisturizing liquid was same as that used in Example 1.

The nonwoven fabric before the moisturizing treatment (the pre-moisturized nonwoven fabric) was employed as Comparative Example 7.

Table 5 shows the results of property evaluation tests and sensory tests of Example 7 and Comparative Example 7. The property evaluation tests and the sensory tests for Example and Comparative Example were carried out as follows.

(Example 8)*

The moisturizing liquid was applied to a commercialized cooking sheet (METSUKE 38.1 g/m$^2$) by a gravure coating method. After air dried and moisture conditioning, the obtained moisturized nonwoven fabric was subjected to measurement to find that METSUKE was 55.6 g/m$^2$. The moisturizing liquid was same as that used in Example 1.

The nonwoven fabric before the moisturizing treatment (the pre-moisturized nonwoven fabric) was employed as Comparative Example 8.

Table 5 shows the results of property evaluation tests and sensory tests of Example 8 and Comparative Example 8. The property evaluation tests and the sensory tests for Example and Comparative Example were carried out as follows.

[Table 5]

| | | Example 7 | Comparative Example 7 | Example 8 | Comparative Example 8 |
|---|---|---|---|---|---|
| METSUKE | g/m$^2$ | 74.3 | 51.1 | 55.6 | 38.1 |
| content of water-soluble component | % | 45.4 | 0.0 | 46.2 | 0.0 |
| equiliblium water content | % | 11.4 | 8.6 | 9.2 | 5.4 |
| increase ratio of equiliblium water content | % | 1.8 | 0.0 | 3.8 | 0.0 |
| strength (vertical) | N | 17.4 | 49.3 | 3.7 | 8.0 |
| (transverse) | N | 5.4 | 25.3 | 3.0 | 6.3 |
| elongation (vertical) | % | 21.3 | 22.2 | 20.7 | 17.4 |
| (transverse) | % | 32.7 | 46.1 | 29.2 | 27.5 |
| F5 (vertical) | N | 6.5 | 28.4 | 0.9 | 3.1 |
| (transverse) | N | 1.37 | 6.61 | 0.52 | 1.56 |
| F5/METSUKE (vertical) | N·m$^2$/g | 0.087 | 0.557 | 0.016 | 0.081 |
| Larose method | | | | | |
| 5 second-water absorption/g | ml/g | 0.45 | 3.51 | 0.39 | 0.34 |
| 10 second-water absorption/g | ml/g | 0.83 | 4.41 | 0.69 | 0.64 |
| maximum water absorption/g | ml/g | 3.42 | 6.19 | 3.08 | 4.67 |
| water retention ratio | - | 6.04 | 8.95 | 13.17 | 22.72 |
| cantilever method | | | | | |
| bending resistance/METSUKE (vertical) | mm·m$^2$/g | 0.78 | 1.97 | 0.79 | 2.04 |
| bending resistance/METSUKE (transverse) | mm·m$^2$/g | 0.39 | 0.86 | 0.80 | 1.92 |
| handle O-meter method | | | | | |
| bending resistance/METSUKE (vertical) | mN·m$^2$/g | 2.18 | 8.52 | 1.99 | 7.94 |
| bending resistance/METSUKE (transverse) | mN·m$^2$/g | 0.74 | 1.74 | 1.76 | 5.46 |
| drape coefficient | % | 63 | 83 | 62 | 81 |
| KES surface MIU | - | 0.283 | 0.341 | 0.262 | 0.467 |
| MMD | - | 0.009 | 0.011 | 0.008 | 0.005 |

(continued)

| | | Example 7 | Comparative Example 7 | Example 8 | Comparative Example 8 |
|---|---|---|---|---|---|
| KES bending B (vertical) | gf·cm$^2$/cm | 0.1257 | 0.4360 | 0.0761 | 0.2097 |
| B (transverse) | gf·cm$^2$/cm | 0.0258 | 0.0700 | 0.0565 | 0.1563 |
| KES bending 2HB (vertical) | gf·cm/cm | 0.1395 | 0.4559 | 0.1234 | 0.2842 |
| 2HB (transverse) | gf·cm/cm | 0.0224 | 0.0442 | 0.0797 | 0.1857 |
| KES shear G (vertical) | gf/cm·degree | 1.94 | 4.33 | 0.88 | 1.76 |
| G (transverse) | gf/cm·degree | 2.04 | 4.06 | 0.82 | 1.90 |
| KES shear 2HG (vertical) | gf/cm | 4.43 | 6.99 | 2.22 | 4.43 |
| 2HG (transverse) | gf/cm | 5.84 | 9.25 | 2.29 | 4.79 |
| Qmax | J/cm$^2$/sec | 0.103 | 0.075 | 0.076 | 0.050 |
| sensory tests | | | | | |
| softness | | good | poor | average | poor |
| smoothness | | good | average | average | average |
| Moisture feeling | | good | poor | average | poor |
| skin moisturizing property | | good | poor | average | poor |
| wiping property | | good | average | good | average |

[Property Evaluation Tests] (Embodiment 1)

Each sample of the Examples was tested after a moisture conditioning in environments of 23°C and 50% RH and subjected to evaluation according to JIS P8111 (standardized state for a moisture conditioning and a test for papers, sheets, and pulps).

<METSUKE (weight per unit surface area)>

The weight per unit surface area measured according to JIS L 1913 (Test methods for nonwovens made of staple fibers) is defined as METSUKE.

<Content of water-soluble component>

The content (percentage) of the water-soluble component in the samples was measured according to the following procedure.

Five sheets of each sample prepared by cutting the sample in a size of 100 mm × 100 mm were moisture conditioned in standardized conditions (23°C and 50%) and weighed (the weight was defined as A, that is, A = total weight of the pre-moisturized nonwoven fabric, equilibrium moisture in the pre-moisturized nonwoven fabric, a moisture-retaining component and equilibrium moisture in the moisture-retaining component). The sample sheets were washed in 2 liter of distilled water at 60°C for 10 minutes while being stirred and sufficiently dewatered. (If some of the fibers are depleted, the depleted fibers are filtered using a piece of filter paper. The obtained depleted fibers are processed similarly to the sample sheets of the nonwoven fabric and added to the weight B of the sample sheets of the nonwoven fabric.) Moreover, the water-soluble component was removed from the sample sheets by drying with a hot air dryer at 105°C for three hours. Successively, the dried sheets were again moisture conditioned in standardized conditions (23°C and 50%) and weighed (the weight was defined as B, that is, B = total weight of the pre-moisturized nonwoven fabric and equilibrium moisture in the pre-moisturized nonwoven fabric). The content (percentage) of the water-soluble component was calculated according to the following equation.

$$\text{Water-soluble component content (\%)} = (A - B) / B \times 100$$

<Equilibrium moisture regain>

The measurement was carried out according to the following procedure with reference to JIS P8127 (Paper and board - Determination of moisture content-Oven-drying method).

Five sheets of each sample prepared by cutting the sample in a size of 100 mm × 100 mm were moisture conditioned in standardized conditions (23°C and 50%) and weighed (the weight was defined as A). Each sample sheet was put in a weighing bottle (cylindrical weighing bottle with 60 mm girth and 80 mm height) and closed there and weighed (the weight was defined as B). After a cover of the weighing bottle was opened, the sample sheet was dried with a hot air drier at 105°C for 3 hours. Successively the cover was closed and the sample sheet was cooled to 23°C in a desiccator and weighed (the weight was defined as C). The equilibrium moisture regain was calculated according to the following equation.

$$\text{Equilibrium moisture regain (\%)} = (B - C) / A \times 100$$

<Increase ratio of equilibrium moisture regain>

The difference of the equilibrium moisture regain measured by the above-mentioned method between the moisturized nonwoven fabric and the nonwoven fabric from which the water- soluble component was removed (the pre-moisturized nonwoven fabric) is defined as an increase ratio of equilibrium moisture regain.

$$\text{Increase ratio of equilibrium moisture regain (\%)} = (\text{equilibrium moisture regain of}$$
$$\text{moisturized nonwoven fabric)} - (\text{equilibrium moisture regain of nonwoven fabric from}$$
$$\text{which the water-soluble component was removed)}$$

<Elongation measurement>

The measurement was carried out according to JIS L1913 (Test methods for nonwovens made of staple fibers). The test was carried out in conditions of 50 mm sample width, 100 mm gripping intervals and 300 mm/min pulling speed. F5-value of each sample is the tensile strength measured at an elongation of 5%.

<Water absorption amount>

The water absorption amounts at a moment 5 seconds after starting the test, at a moment 10 seconds after starting, and at a moment of saturation were respectively measured by Larose method. The water absorption amount per 1 g of each sample was calculated by dividing the measured water absorption weight by the sample weight.

<Water retention ratio>

The water retention ratio was measured by the following method.

Each sample cut in a size of 100 mm × 100 mm was moisture conditioned in standardized conditions (23°C and 50%) and weighed (the weight was defined as A). After the sample was immersed in distilled water in a tray for 60 seconds, the sample was left on a metal net while being slanted at a tilting angle of 30° for 60 seconds and quickly subjected to weight measurement (the weight was defined as B). The water retention ratio was calculated according to the following equation.

$$\text{Water retention ratio} = (B - A) / A$$

<Bending resistance>

Bending resistance standardized in JIS L1096 (Testing methods for woven fabrics) was measured by A method (45° cantilever method), E method (handle O meter method), and G method (drape coefficient). In the E method, the sample size was 100 mm $\times$ 100 mm and measurement was carried out at the center of each sample. The slit width was set to be 10 mm.

<KES surface test>

The measurement was carried out using KES SE friction tester manufactured by Kato Tech Co., Ltd. A friction element (Silicone Sensor for KES SE, manufactured by Kato Tech Co., Ltd.) coated with silicon rubber was employed. The measurement was carried out for the front face and the rear face of each sample in vertical direction and transverse direction in conditions of 1 cm$^2$ contact surface area of the friction element; 25 gf/cm$^2$ load, and 1 mm/sec sample stand moving speed. The average of all measured values was employed.

<KES pure bending test>

Bending rigidity B and hysteresis of bending moment 2HB were measured by KES FB 2 pure bending tester manufactured by Kato Tech Co., Ltd. The measurement was carried out in conditions of 200 mm sample width and standardized high sensitivity. If the sample size was smaller than 200 mm, the measurement may be carried out with 100 mm sample width.

<KES shear test>

The shear stiffness G and the hysteresis of shear force at 0.5° of shear angle 2HG were measured by KES FB 1 shear tester manufactured by Kato Tech Co., Ltd. The measurement was carried out in conditions of 200 mm sample width and standardized high sensitivity.

<The paek value of the heat flow Qmax>

Qmax was measured by KES F7 TERHMO LABO manufactured by Kato Tech Co., Ltd. The measurement was carried out at a sample temperature of 20°C, a copper plate initial temperature of 3 0°C, and a contact pressure of 10 gf/cm$^2$. The measurement was carried out for the front face and the rear face of each sample and the average of the measured values was employed.

[Sensory tests]

The following items were evaluated by touching each sample with hands of 10 panelists.

(Softness)

Evaluation was carried out by calculating the total points in the following 4-point evaluation system: very soft = 4 points; soft = 3 points; slightly soft = 2 points; and not soft = 1 point.
The evaluation was carried out according to the following standard: "Very good" for 36-40 points; "Good" for 26 to 35 points; "Average" for 16 to 25 points; and "Poor" for 10 to 15 points.
The following respective items were evaluated in the same manner by calculating the total points and carrying out evaluation according to the similar standards.

(Smoothness)

Very smooth = 4 points; smooth = 3 points; slightly smooth = 2 points; and not smooth = 1 point

(Moist feeling)

Very moist = 4 points; moist = 3 points; slightly moist = 2 points; and not moist = 1 point

(Skin moisture retention)

The evaluation was performed on moisture felt in the skin when wiping hands with each sample sheet after washing the hands with water.
Strongly felt moist in skin = 4 points; felt moist in skin = 3 points; slightly felt moist in skin = 2 points; and not felt moist in skin = 1 point

(Stain wiping property for skin)

The test was carried out assuming that stain around the eye is gently wiped so as not to irritate the eye. A circle of 1 cm in diameter is drawn with a marker in the middle of the palm assuming that the circle is an eye, and ketchup was applied to all over the palm. The evaluation was performed on the ease of wiping when ketchup outside the circle is wiped with each sample with care so as not to touch the ketchup in the circle.
Very good wiping property = 4 points; good wiping property = 3 points; slightly good wiping property = 2 points; and inferior wiping property = 1 point

[Results of tests]

As compared with nonwoven fabrics which were not subjected to moisturizing treatment, all of the nonwoven fabrics subjected to the moisturizing treatment were found having significantly improved properties of softness, smoothness, moist feeling, and the skin moisture retention. With respect to the stain wiping property for skin, although stains were removed even with the nonwoven fabrics of Comparative Examples, delicate work is difficult with the rough fabrics since they were inferior in smoothness and softness. Also, the physical irritation to the skin became significant.
Sample sheets with different amounts of the moisturizing liquid were produced in Examples 1 and 2. The sheets of Example 1-1 and Example 2-1 were sufficiently soft and gave dry feeling rather than moist feeling. As the application amount of the moisturizing liquid was increased, the moist feeling was more intensified. The sample sheets of Example 1-3 and Example 2-3 gave noticeable moisture retention feeling for the skin.
In the case the moisturizing treatment was carried out even for the nonwoven fabric produced by intense hydroentangling treatment just like the case of Example 3, the obtained moisturized nonwoven fabric had a pleasant texture.
In the case the moisturizing treatment was carried out even for the nonwoven fabric containing a large amount of pulp just like the case of Example 4, the nonwoven fabric was excellent in especially moist feeling and the nonwoven fabric also showed excellent water absorption as well.
In the case the moisturizing treatment was carried out even for the nonwoven fabric containing no pulp just like the case of Example 5, the obtained moisturized nonwoven fabric was provided with further improved softness and also provided with moist feeling.
In the case the moisturizing treatment was carried out even for the nonwoven fabrics produced in wetlaid method just like the case of Examples 6 and 7, the obtained moisturized nonwoven fabrics had a pleasant texture.
In the case the moisturizing treatment was carried out even for the nonwoven fabric produced in the airlaid method just like the case of Example 8, the obtained moisturized nonwoven fabrics showed a progress in softness, moist feeling, and skin moisture retention.

[Embodiment 2]

(Example 9)

Softwood kraft pulp (Howe Sound 400, manufactured by Canfor Corporation) was beaten and disintegrated to Canadian freeness of 680 ml to produce pulp paper with 32 g/m$^2$ by a cylinder type paper manufacture machine.
Mixed fibers containing 50% by weight of rayon fibers (fineness 1.7 dtex $\times$ fiber length 40 mm) and 50% by weight of PET fibers (fineness 0.9 dtex $\times$ fiber length 44 mm) were fibrillated by a carding machine to produce fiber webs. METSUKE of a sheet of the fiber webs was controlled to be 14 g/m$^2$.
The above-mentioned fiber webs was laid on the pulp paper and fed to a conveyer belt. The conveyer belt was made of a plastic net with 50 meshes and moved at about 7 m/min.
The pulp paper and the webs were subjected to entangling and uniting treatment by a water jet treatment apparatus installed on the path of the conveyer belt. The high pressure columnar current for the entangling treatment was generated using water as a fluid. Water jet machines each having nozzles with a diameter of 0.08 mm and arranged at 1 mm pitches in the transverse direction were arranged in three rows perpendicularly to the moving direction of the conveyer belt. The distance between the nozzles of the water jet machines and the surface of the layered sheet was set to be 2 cm. Water current at pressure of 2 MPa, 4 MPa, and 4 MPa was jetted from the three rows of the water jet machines. Successively, the hydroentangling treatment was carried out for the rear face of the layered sheet in the same conditions. The layered sheet for which the water jet treatment was finished was successively subjected to dewatering treatment and drying treatment. The obtained nonwoven fabric had 45 g/m$^2$ METSUKE.
Then, the nonwoven fabric was moisturized. A moisturizing liquid was applied to the nonwoven fabric by a gravure coating method. After air drying and moisture conditioning, the obtained moisturized nonwoven fabric was subjected to measurement to find that METSUKE was 58.5 g/m$^2$. That is, the content of the water-soluble component was 30%.
The moisturizing liquid was produced by mixing 60% by weight of glycerin, 10% by weight of sorbitol, 1% by weight of decaglycerin monostearic acid ester, 5% by weight of liquid paraffin, 1% by weight of polyoxyethylene (20 EO) sorbitan monostearate, 0.4% by weight of sorbitan monostearate, and 22.6% by weight of water.

(Example 10)

Softwood kraft pulp (Howe Sound 400, manufactured by Canfor Corporation) was beaten and disintegrated to Canadian freeness of 680 ml to produce pulp paper with 20 g/m$^2$ by a cylinder type paper manufacture machine.
Mixed fibers containing 80% by weight of rayon fibers (fineness 1.7 dtex $\times$ fiber length 40 mm) and 20% by weight of PET fibers (fineness 0.9 dtex $\times$ fiber length 44 mm) were fibrillated by two carding machines to produce a pair of fiber webs. METSUKE of a sheet of the fiber webs was controlled to be 10 g/m$^2$.
The above-mentioned pulp paper was sandwiched between the pair of fiber webs and fed to a conveyer belt. The conveyer belt was made of a plastic net with 20 meshes and moved at about 7 m/min.
The pulp paper and the webs were subjected to entangling treatment by a water jet treatment apparatus installed on the path of the conveyer belt, using high pressure columnar current. The high pressure columnar current was generated using water as a fluid. Water jet machines each having nozzles with a diameter of 0.08 mm and arranged at 1 mm pitches in the transverse direction were arranged in three rows perpendicularly to the moving direction of the conveyer belt. The distance between the nozzles of the water jet machines and the surface of the layered sheet was set to be 2 cm. Water current at pressure of 2 MPa, 4 MPa, and 4 MPa was jetted from the three rows of the water jet machines. Successively, the hydroentangling treatment was carried out for the rear face of the layered sheet in the same conditions. The layered sheet for which the water jet treatment was finished was successively subjected to dewatering treatment and drying treatment. The obtained nonwoven fabric had 40 g/m$^2$ METSUKE.
Then, the nonwoven fabric was moisturized. The moisturizing liquid same as that used in Example 9 was applied to the nonwoven fabric by a gravure coating method. After air drying and moisture conditioning, the obtained moisturized nonwoven fabric was subjected to measurement to find that METSUKE was 72 g/m$^2$. That is, the content of the water-soluble component was 80%.

(Example 11)

Softwood kraft pulp (Howe Sound 400, manufactured by Canfor Corporation) was disintegrated by a pulper to produce pulp paper with 25 g/m$^2$ by a cylinder type paper manufacture machine. In this case, no beating treatment was carried out.
Rayon fibers (fineness 1.7 dtex $\times$ fiber length 40 mm) were fibrillated by two carding machines to produce a pair of fiber webs. METSUKE of a sheet of the fiber webs was controlled to be 13 g/m$^2$.

The above-mentioned pulp paper was sandwiched between two sheets of the fiber webs and fed to a conveyer belt. The conveyer belt was made of a plastic net with 50 meshes and moved at about 7 m/min.

The pulp paper and the webs were subjected to entangling treatment by a water jet treatment apparatus installed on the path of the conveyer belt, using high pressure columnar current. The high pressure columnar current was generated using water as a fluid. Water jet machines each having nozzles with a diameter of 0.08 mm and arranged at 1 mm pitches in the transverse direction were arranged in three rows perpendicularly to the moving direction of the conveyer belt. The distance between the nozzles of the water jet machines and the surface of the layered sheet was set to be 2 cm. Water current at pressure of 3 MPa, 6 MPa, and 6 MPa was jetted from the three rows of the water jet machines. Successively, the hydroentangling treatment was carried out for the rear face of the layered sheet in the same conditions. The layered sheet for which the water jet treatment was finished was successively subjected to dewatering treatment and drying treatment. The obtained nonwoven fabric had 50 g/m$^2$ METSUKE.

Then, the nonwoven fabric was moisturized. The moisturizing liquid same as that used in Example 1 was applied to the nonwoven fabric by a gravure coating method. After air drying and moisture conditioning, the obtained moisturized nonwoven fabric was subjected to measurement to find that METSUKE was 60 g/m$^2$. That is, the content of the water-soluble component was 20%.

[Comparative Examples 9 to 11]

The nonwoven fabrics of the comparative Examples 9 to 11 were same as those of Examples 9 to 11 except that the nonwoven fabric were not subjected to moisturizing treatment and therefore they shows the properties of the raw nonwoven fabrics themselves.

[Property Evaluation Tests] (Embodiment 2)

<METSUKE, Density>

The weight per unit surface area measured according to JIS L1913 (Test methods for nonwovens made of staple fibers) is defined as METSUKE.

Using the following equation, the density was calculated from the thickness measured similarly according to JIS L 1913. The ambient conditions were controlled to a standardized state (temperature of 23°C and humidity of 50% RH) according to JIS P8111 (Paper, board and pulps - Standard atmosphere for conditioning and testing).

$$\text{Density (g/cm}^3) = \text{METSUKE (g/m}^2) / \text{thickness (}\mu\text{m)}$$

<Water-soluble component content>

The water-soluble component content is the increase ratio of METSUKE (density) due to the application of the moisturizing liquid and water to each nonwoven fabric in the moisturizing treatment.

As the water-soluble component content is higher, it means that the retention amount of the water-soluble components is higher. As the application amount of the moisturizing liquid is increased, the water-soluble component content is increased, however, since there are components which are evaporated and diffused after the moisturizing treatment and on the contrary, water may be taken in the water-soluble components from atmospheric air, the coating amount of the moisturizing liquid and the water-soluble component content are not necessarily accurately coincident with each other.

In the case A was defined as METSUKE of a nonwoven fabric before the moisturizing treatment measured according to JIS-L1913 and B was defined as METSUKE of the nonwoven fabric after the moisturizing treatment measured again according to JIS-L1913, the moisturizing liquid treatment ratio was calculated according to the following equation. The measurement conditions were same as the standardized state according to JIS-P8127 (23°C and 50% RH).

$$\text{Water-soluble component content (\%)} = (B - A) / A \times 100$$

<Water activity value Aw>

The water activity value Aw of the nonwoven fabric is preferably 0.7 or lower in the standardized state (23°C and 50% RH). If it is 0.7 or lower, propagation of mould and microorganism can be suppressed and thus there is no need to use a preservative or an anti-mold agent. For this purpose, it is preferable to add glycerin as a moisturizing component.

In the above-mentioned Examples, the water activity value was measured by a water activity meter (an electric resistance type hygrometer).

<Drape coefficient>

As the drape coefficient is smaller, the nonwoven fabric more fits with a hand of a user or the shape of an object to be wiped when the nonwoven fabric is used as a wiper. If the drape coefficient is high, the nonwoven fabric becomes stiff and uneasy to use.

In the above-mentioned Examples, the drape coefficient was measured by a drape tester (YD-100 model, manufactured by Daiei Kagaku Seiki Seisakusho) and calculated according to the following equation.

$$\text{Drape coefficient (\%)} = [(Ad - S1) / (S2 - S1)] \times 100$$

Ad: perpendicular projection surface area of sample (draping surface area)
S1: surface area of a sample stand (diameter 12.7 cm)
S2: surface area of a sample (diameter 25.4 cm)

<The paek value of the heat flow Qmax>

The paek value of the heat flow Qmax is measured as heat quantity fluxing at the moment of contact. If Qmax is high, it is felt cold at the moment of contact and if it is low, it is felt warm.

In the above-mentioned Examples, a measurement apparatus (KES F7 THERMO LABO II, manufactured by Kato Tech Co., Ltd.) was used to measure Qmax (the paek value of the heat flow). The measurement was carried out at a sample temperature of 20°C; the copper plate initial temperature of 30°C; and contact pressure of 10 gf/cm$^2$.

<Elongation measurement>

If the breaking tenacity is too low, the nonwoven fabric may be torn at the time of use. If it is too high, the nonwoven fabric gives stiff feeling. The fracture elongation is preferably in a range from 10 to 150% in both vertical and transverse directions. If the fracture elongation is too low, the nonwoven fabric may be torn at the time of use and may not be deformed into the proper shape of the object to be wiped and thus becomes inconvenient for use.

In Examples, the measurement was carried out according to JIS L1913. The test was carried out in conditions of 100 mm gripping intervals and 300 mm/min pulling speed. MD/CD means the measured values in vertical direction/ transverse direction, respectively.

<KES B>

The B value of Kawabata type feeling measurement value KES-FB 2 shows the bending stiffness (tilting at a curvature of 1 cm$^{-1}$ in the bending test), and as the B value is higher, the bending stiffness is high and as it is lower, it means soft for bending.

The measurement was carried out by KES FB 2 pure bending tester manufactured by Kato Tech Co., Ltd. The measurement was carried out in conditions of 200 mm sample width and standardized high sensitivity. MD/CD means the same as that in elongation measurement.

<Water absorption capability>

The water absorption capability is preferably 0.1 ml or higher by saturated water absorption measurement by Larose method. If the water absorption capability is low, a large quantity of water remains after wiping in the case of using the nonwoven fabric as a wiper and therefore, it is not preferable.

In Examples, the saturated water absorption amount was measured by conventionally known Larose method.

[Sensory test]

The following items were evaluated by touching each sample with hands of ten panelists.

(Softness)

Evaluation was carried out by calculating the total points in the following 4-point evaluation system: very soft = 4 points; soft = 3 points; slightly soft = 2 points; and not soft = 1 point.

The evaluation was carried out according to the following standard: "Very good" for 36-40 points; "Good" for 26 to 35 points; "Average" for 16 to 25 points; and "Poor" for 10 to 15 points.

The following respective items were evaluated in the same manner by calculating the total points and carrying out evaluation according to the similar standards.

(Smoothness)

Very smooth = 4 points; smooth = 3 points; slightly smooth = 2 points; and not smooth = 1 point

(Moist feeling)

Very moist = 4 points; moist = 3 points; slightly moist = 2 points; and not moist = 1 point

(Skin moisture retention)

Strongly felt moist in skin = 4 points; felt moist in skin = 3 points; slightly felt moist in skin = 2 points; and not felt moist in skin = 1 point

[Results of tests]

[Table 6]

<Effect of moisturizing treatment>

| | | Examples | | | Comparative Examples | | |
|---|---|---|---|---|---|---|---|
| | | 9 | 10 | 11 | 9 | 10 | 11 |
| fiber composition: | wt% | | | | | | |
| pulp | | 70 | 50 | 50 | 70 | 50 | 50 |
| rayon | | 15 | 40 | 50 | 15 | 40 | 50 |
| PET | | 15 | 10 | 0 | 15 | 10 | 0 |
| production conditions: | | | | | | | |
| conveyer belt | mesh | 50 | 20 | 50 | 50 | 20 | 50 |
| highest pressure | MPa | 4 | 4 | 6 | 4 | 4 | 66 |
| METSUKE | g/m$^2$ | 45 | 40 | 50 | 45 | 40 | 50 |
| moisturizing ratio % | treatment | 30 | 80 | 20 | 0 | 0 | 0 |
| nonwoven fabric properties: | | | | | | | |
| density | g/cm$^3$ | 0.15 | 0.15 | 0.14 | 0.10 | 0.10 | 0.11 |
| drape coefficient | % | 43.7 | 42.3 | 59.9 | 75.6 | 72.1 | 80.5 |
| Qmax | J/cm$^2$/s | 0.12 | 0.10 | 0.09 | 0.08 | 0.08 | 0.08 |
| breaking tenacity | MD/CD N | 20/5 | 22/7 | 25/10 | 35/7 | 45/15 | 55/25 |
| fracture elongation | MD/CD % | 24/59 | 45/118 | 43/75 | 19/55 | 28/71 | 23/65 |
| KES B | MD/CD | 0.030 | 0.050 | 0.075 | 0.250 | 0.180 | 0.340 |
| | gfcm$^2$/cm | /0.005 | /0.003 | /0.014 | /0.015 | /0.015 | /0.029 |
| water absorption ml | capability | 0.33 | 0.18 | 0.60 | 0.88 | 0.67 | 0.83 |

(continued)

<Effect of moisturizing treatment>

| | Examples | | | Comparative Examples | | |
|---|---|---|---|---|---|---|
| | 9 | 10 | 11 | 9 | 10 | 11 |
| sensory tests: | | | | | | |
| softness | Very good | Very good | good | poor | poor | poor |
| smoothness | good | Very good | good | poor | poor | poor |
| moisture feeling | good | Very good | good | poor | poor | poor |
| skin moisture retention | good | Very good | good | poor | poor | poor |

[Evaluation]

(1) As compared with Comparative Examples 1 to 3 which were not subjected to the moisturizing treatment, Examples 9 to 11 subjected to the moisturizing treatment were found significantly excellent in the softness, smoothness, moist feeling, and skin moisture retention in the sensory test.

This can be understood by comparing the respective property values. The drape ratios of the nonwoven fabrics of Examples are small, showing that the nonwoven fabrics are easy to fit with hands and objects. The Qmax values are not so much different from those of Comparative Examples and the nonwoven fabrics of Examples are not felt unpleasantly cold even if they are brought into contact with the skin. KES B values are small and it means that the nonwoven fabrics are soft and flexible. Although the breaking tenacity values of Examples are smaller than those of Comparative Examples, the nonwoven fabrics of Examples have sufficient strength for practical use. The fracture elongation of the moisturized nonwoven fabrics of Examples is higher in all cases as compared with that of the nonwoven fabrics of Comparative Examples with the same number. The water absorption capability is lower than that of Comparative Examples. It is because the nonwoven fabrics of Examples already retain water.

(2) Comparing Examples 9 to 11 with each other, it can be understood that the properties and capabilities of the moisturized nonwoven fabrics are made different depending on the combination of fibers, treatment conditions of the hydroentangling treatment, and the like.

[Moisturizing treatment ratio and capability]

The relation between the moisturizing treatment ratio and capability was investigated.

(Examples 12 to 14)

The same nonwoven fabric was used as used in Example 9 and the nonwoven fabric was moisturized by the same manner as that of the Example 9, but the moisturizing treatment ratio was changed by changing the application amount of the moisturizing liquid.

<Test results>

[Table 7]

<Moisturizing treatment ratio and capability>

| | | Examples | | |
|---|---|---|---|---|
| | | 12 | 13 | 14 |
| moisturizing treatment ratio | % | 200 | 80 | 10 |
| nonwoven fabric properties: | | | | |
| density | g/cm$^3$ | 0.33 | 0.20 | 0.14 |
| drape coefficient | % | 28.7 | 44.9 | 62.5 |
| Qmax | J/cm$^2$/s | 0.18 | 0.14 | 0.10 |
| breaking MD/CD N | tenacity | 17/3 | 18/4 | 21/6 |

(continued)
<Moisturizing treatment ratio and capability>

| | | | Examples | | |
|---|---|---|---|---|---|
| | | | 12 | 13 | 14 |
| | fracture elongation | MD/CD % | 30/120 | 27/104 | 23/67 |
| | KES B | MD/CD | 0.028 | 0.033 | 0.093 |
| | | gfcm$^2$/cm | /0.004 | /0.005 | /0.013 |
| | water absorption ml | capability | 0.17 | 0.20 | 0.71 |
| sensory tests: | | | | | |
| | softness | | Very good | Very good | good |
| | smoothness | | poor | Very good | poor |
| | moisture feeling | | Very poor | Very good | poor |
| | skin moisture retention | | Very good | Very good | average |

[Evaluation]

(1) As the moisturizing treatment ratio, that is the amount of the water-soluble component, is increased from Example 14 to Example 12, the drape ratio is lowered and softness is increased. Since Qmax is increased, it is felt cold but not felt uncomfortable. KES B value is decreased to make bending easy.

(2) It is understood that the moisturized nonwoven fabric of Example 13 containing 80% by weight of the water-soluble component is better in comprehensive aspects than the moisturized nonwoven fabric of Example 14 containing 10% by weight of the water-soluble component.

[Preparation of moisturizing liquid]

The effects on the physical values and the sensory test were investigated by changing the addition amounts of components of the moisturizing liquid.

(Examples 15 to 19)

The moisturizing treatment was carried out for the same nonwoven fabric of Example 11 in the same manner as in Example 11, except that the addition amounts of components of the moisturizing liquid were changed.

<Test results>

[Table 8]
<Moisturizing liquid composition and properties>

| | Examples | | | | |
|---|---|---|---|---|---|
| | 15 | 16 | 17 | 18 | 19 |
| moisturizing liquid composition: % | | | | | |
| glycerin | 70 | 60 | 60 | 60 | 60 |
| sorbitol | - | 10 | 10 | 10 | 10 |
| decaglycerin monostearic acid ester | - | - | 1 | 1 | 1 |
| liquid paraffin | - | - | - | 5 | 5 |
| dimethylpolysiloxane (100 cs) | - | - | - | - | 1 |
| polyoxyethylene (20 EO) sorbitan monostearate | - | - | - | 1 | 1 |
| sorbitan monostearate | - | - | - | 0.4 | 0.4 |
| water | 30.0 | 30.0 | 29.0 | 22.6 | 21.6 |
| moisturizing treatment ratio % | 20 | 20 | 20 | 20 | 20 |

(continued)

<Moisturizing liquid composition and properties>

| | Examples | | | | |
|---|---|---|---|---|---|
| | 15 | 16 | 17 | 18 | 19 |
| nonwoven fabric properties: | | | | | |
| density g/cm$^3$ | 0.12 | 0.12 | 0.12 | 0.12 | 0.12 |
| drape coefficient % | 68.8 | 65.6 | 62.3 | 59.3 | 56.5 |
| Qmax J/cm$^2$/s | 0.10 | 0.10 | 0.10 | 0.11 | 0.11 |
| breaking tenacity MD/CD N | 21/6 | 20/5 | 20/5 | 20/5 | 20/5 |
| fracture elongation MD/CD % | 26/69 | 25/73 | 27/67 | 28/64 | 25/55 |
| KES B MD/CD | 0.078 | 0.065 | 0.052 | 0.049 | 0.039 |
| gfcm$^2$/cm | /0.013 | /0.011 | /0.009 | /0.008 | /0.007 |
| water absorption capability ml | 0.75 | 0.75 | 0.45 | 0.48 | 0.50 |
| sensory tests: | | | | | |
| softness | average | good | good | Very good | Very good |
| smoothness | average | average | good | good | Very good |
| moisture feeling | average | average | average | good | good |
| skin moisture retention | good | good | good | good | good |

<Evaluation>

(1) All of the moisturizing liquids employed for Examples 15 to 19 are found efficacious, however it is understood the respective properties and the evaluations by the sensory test differ depending on the compositions of the moisturizing liquids.

(2) In the case sorbitol is added in addition to glycerin as a moisturizing agent, the softness is improved (Examples 16 to 19). In the case a glycerin fatty acid ester is added, the smoothness is improved (Examples 17 to 19). In the case oils are added, the softness and the moist feeling are improved (Examples 18 to 19). In the case silicones are added, smoothness is further improved (Example 1 19).

[Comparison with commercialized products: wet tissues]

Properties were compared with those of commercialized wet tissue products.

(Comparative Example 12)

Commercialized wet tissues (manufactured by Sanshoshigyo Co., Ltd.) were used.

[Evaluation tests]

Drying property: the respective samples were left in an atmosphere of constant temperature and constant humidity (23°C and 50% RH) for 24 hours and the weight change was observed. The wet tissues were taken out of the container and immediately subjected to initial weight measurement.

Stimulus property: similarly to the above-mentioned sensory test, whether the skin was irritated or not was evaluated by the panelists by touching the samples.

Tests other than these tests were carried out similarly to the above-mentioned manner.

[Test results]

[Table 9]

<Comparison with commercialized products: wet tissues>

|  |  | Example | Comparative Example |
|---|---|---|---|
|  |  | 9 | 12 |
| drying property: |  |  |  |
|  | initial weight A g | 5.00 | 5.00 |
|  | weight after being left B g | 5.00 | 1.50 |
|  | difference (A - B)/B % | 0 | 233 |
| irritation |  | no | yes |
| Qmax | $J/cm^2/s$ | 0.11 | 0.37 |
| skin moisture retention feeling |  | good | poor |
| water activity value |  | 0.50 | 0.97 |

[Evaluation]

(1) The commercialized wet tissues of Comparative Example 12 have significant drying property and cannot be stored unless the storage is in an air-tight condition. The moisturized nonwoven fabric of Example 9 does not have drying property, there is no problem even if the moisturized nonwoven fabric is kept in an open state.

(2) It was proved that, as compared with the wet tissues of Comparative Example 12, the moisturized nonwoven fabric of Example 9 less irritates the skin, gives no uncomfortable cold feeling (Qmax is small), is excellent in the moisture retention feeling, and requires no preservative or the like (small water activity value).

[Comparison with commercialized products: moisturized tissues]

Properties were compared with those of commercialized moisturizing tissue products.

(Comparative Example 13)

Commercialized moisturized tissues (trade name: "Uruoi Hoshitsu", manufactured by KAWANO PAPER Co., Ltd.) were used. Each tissue is two-ply paper produced by laminating two thin pulp paper sheets. The pulp used for the pulp paper does not contain fibers with a length exceeding 20 mm.

[Evaluation tests]

Water retention amount: Samples were immersed in water and left on a metal net tilted at 45° and the water weight was measured. The samples were left for 5 minutes.

Wiping property: stains were applied to the back of a hand and the remaining amounts were compared by eye observation when the stains were wiped using the samples. Ketchup was used for the stains. When the stains were sufficiently wiped off, it is given "good" evaluation. When the stains were not sufficiently wiped off, it is given "poor" evaluation.

METSUKE, tensile strength, and water absorption capability were measured according to JIS S3104. For Example 1, a test sample with a width of 50 mm was used, meanwhile two samples with a width of 25 mm were combined for the measurement in Comparative Example 5.

The test items other than these items were measured in the same manner as described above or by conventional methods.

[Test results]

[Table 10]

<Comparison with commercialized moisturized tissues>

|  | Example | Comparative Example |
|---|---|---|
|  | 9 | 13 |
| physical properties: |  |  |
| ply sheet | 1 | 2 |
| METSUKE g/m$^2$ | 58.5 | 37.6 |
| thickness mm | 0.39 | 0.16 |
| density g/cm$^3$ | 0.15 | 0.24 |
| tensile strength MD/CD N | 20/5 | 3/0.9 |
| elongation MD/CD % | 24/59 | 14/6 |
| water absorption capabilitys | 0.6 | 5.0 |
| water retention amount g/m$^2$ | 420 | 145 |
| sensory tests: |  |  |
| softness | Very good | average |
| smoothness | good | average |
| moisture feeling | good | average |
| skin moisture retention | good | average |
| wiping property | good | poor |

[Evaluation]

(1) As compared with commercialized moisturized tissues of Comparative Example 13, the moisturized non-woven fabric of Example 9 has high water retention amount, gives good skin touch and is excellent in the wiping property, as well as being excellent in strength.

This proves the technical advantage of the invention of using the nonwoven fabric containing fibers with a length of 20 mm or longer.

[Comparison with commercialized products: using commercialized nonwoven fabrics]

Properties of the moisturized nonwoven fabric of Example 9 were compared with those of commercialized nonwoven fabric products subjected to the same moisturizing treatment as Example 9.

(Comparative Example 14)

A commercialized pulp nonwoven fabric (trade name: "Neoranu", manufactured by KUREHA CORPORATION) produced by the airlaid method was used. No fiber with a fiber length of 20 mm or longer was contained.

(Comparative Example 15)

A commercialized wetlaid spunbonded nonwoven fabric (trade name: "Taiko TCF #503", manufactured by Futamura Chemical Co., Ltd., fiber length: 10 mm) produced from rayon short fibers by wetlaid method was used. No fiber with a fiber length of 20 mm or longer was contained.

[Test results]

[Table 11]

<Comparison with commercialized nonwoven fabrics>

|  | Example | Comparative | Examples |
|---|---|---|---|
|  | 9 | 14 | 15 |
| fibers with length of 20 mm or longer % | 30 | 0 | 0 |

(continued)

<Comparison with commercialized nonwoven fabrics>

| | Example | Comparative | Examples |
|---|---|---|---|
| | 9 | 14 | 15 |
| moisturizing treatment ratio % | 30 | 30 | 30 |
| METSUKE g/m$^2$ | 58.5 | 65 | 33 |
| density g/cm$^3$ | 0.15 | 0.09 | 0.11 |
| breaking tenacity MD/CD N | 20/5 | 9/7 | 9/13 |
| fracture elongation MD/CD % | 24/59 | 18/23 | 14/33 |
| drape coefficient % | 43.7 | 81.2 | 72.0 |
| sensory tests: | | | |
| softness | Very good | poor | poor |
| smoothness | good | average | poor |
| moisture feeling | good | average | average |
| skin moisture retention | good | poor | average |
| Wiping property | good | poor | poor |

[Evaluation]

(1) Comparative Examples 14 and 15 using the commercialized nonwoven fabrics were not improved in the moisture retention function even if the moisturizing treatment was carried out. It proved the technical advantage of the invention of using the specified nonwoven fabric.

(2) The moisturized nonwoven fabric of Example 9 was proved to have significantly improved properties in the sense of use such as the skin touch as compared with those of Comparative Examples 14 and 15.

(3) Comparative Example 14 (using the nonwoven fabric containing airlaid pulp in which fibers are fixed by chemical bonding) was inferior in the drape property. Actually, in the case it was used as a wiper, it gave stiff feeling and was difficult to deform along with the shape of the object to be wiped, and thus inferior in the sense of use.

(4) With respect to Comparative Example 15 (using the nonwoven fabric produced from rayon staple fibers by wetlaid spun bonding), the nonwoven fabric was still felt thin and hard in touching even after the moisturizing treatment and it was thus inferior in the sense of use.

**Claims**

1. A moisturized non-woven fabric made by applying a moisturizing liquid including a water-soluble component, containing one or more moisture-retaining component(s), selected from glycerin, diglycerin, polyglycerin, ethylene glycol, diethylene glycol, polyethylene glycol, propylene glycol, 1,3-butylene glycol, sorbitol, xylitol, erythritol, mannitol, lactitol, oligosaccharide alcohol, maltitol, reduced starch hydrolysis product, fructose, glucose, oligosaccharide, trehalose, glycine betaine, pyrrolidonecarboxylic acid, pyrrolidone carboxylic acid salt, hyaluronic acid, hyaluronic acid salt, lactic acid, lactic acid salt, and urea, and water to a raw non-woven fabric through a moisturizing treatment, wherein the raw non-woven fabric comprises 20 to 80 % by weight of fibers with a length of 20 to 100 mm and a fineness of 0.5 to 10.0 dtex and 20 to 80 % by weight of natural cellulose fibers with a length shorter than 20 mm and is spunlaced, wherein the amount of the water-soluble component is 5 to 150 % to the weight of the raw non-woven fabric; and wherein the increase ratio of the equilibrium moisture regain of the moisturized non-woven fabric is 0.5 to 40 % as compared with an equilibrium moisture regain of the moisturized nonwoven fabric after the water soluble component is removed, measured according to the procedure of JIS P8127 (Paper and board - Determination of moisture content - Oven-drying method).

2. The moisturized non-woven fabric according to claim 1, wherein the F5-value (tensile strength measured at an elongation of 5 %) per unit METSUKE (weight per unit surface area) is 0.40 N.m$^2$/g or lower in the strongest direction, and the strength is 1.0 N or higher in the weakest direction.

3. The moisturized non-woven fabric according to claim 1, wherein the water absorption capacity per 1 g of the moisturized non-woven fabric after 5 seconds from the starting point of measurement by the Larose method is 0.03 to 2.50 ml/g, and the water retention ratio is 3.0 or higher.

4. The moisturized non-woven fabric according to claim 1, wherein the bending resistance per unit METSUKE (weight per unit surface area), measured by a handle O meter, is 5.0 mN·m$^2$/g or lower in the highest bending resistance direction.

5. The moisturized non-woven fabric according to claim 1, wherein the bending resistance per unit METSUKE (weight per unit surface area), measured by the cantilever method, is 1.5 mm·m$^2$/g in the highest bending resistance direction.

6. The moisturized non-woven fabric according to claim 1, wherein the MIU value (friction coefficient value), measured by a friction sensitivity tester, is 0.45 or lower.

7. The moisturized non-woven fabric according to claim 1, wherein the peak value of the heat flow Qmax is 0.08 to 0.30 J/cm$^2$/sec.

8. The moisturized non-woven fabric according to claim 1, comprised of at least one of natural cellulose fibers including wood pulp, regenerated fibers including rayon fibers, and synthetic fibers including polyethylene terephthalate fibers and polypropylene fibers.

9. The moisturized non-woven fabric according to claim 1, comprised of a non-woven fabric obtained by combining fiber webs comprised of fibers with a length of 20 to 100 mm and pulp paper obtained by wet-laid method from natural cellulose fibers with a length shorter than 20 mm in a hydro-entangling process.

10. The moisturized non-woven fabric according to claim 1, wherein the water-soluble component comprises glycerin.

11. The moisturized non-woven fabric according to claim 1, further comprising an additive component selected from oils, fatty acids, higher alcohols, silicones, and waxes.

**Patentansprüche**

1. Ein befeuchtetes Faservlies, hergestellt durch das Auftragen einer befeuchtenden Flüssigkeit, einschließend eine wasserlösliche Komponente, enthaltend eine oder mehrere feuchtigkeitszurückhaltende Komponente(n), gewählt aus Glycerin, Diglycerin, Polyglycerin, Ethylenglykol, Diethylenglykol, Polyethylenglykol, Propylenglykol, 1,3-Butylenglykol, Sorbitol, Xylitol, Erythritol, Mannitol, Lactitol, Oligosaccharidalkohol, Maltitol, Reduzierte-Stärke-Hydrolyseprodukt, Fruktose, Glukose, Oligosaccharide, Trehalose, Glycin, Betain, Pyrrolidoncarboxylsäure, Pyrrolidoncarboxylsäuresalz, Hyaluronsäure, Hyaluronsäuresalz, Milchsäure, Milchsäuresalz und Harnstoff und Wasser auf ein Rohfaservlies durch eine befeuchtende Behandlung, worin das Rohfaservlies 20 bis 80 Gewichts-% an Fasern mit einer Länge von 20 bis 100 mm und einer Feine von 0,5 bis 10,0 dtex und 20 bis 80 Gewichts-% an natürlichen Zellulosefasern mit einer Länge kürzer als 20 mm umfasst und wasserstrahlverfestigt [spunlaced] ist, worin die Menge an wasserlöslicher Komponente 5 bis 150 % von dem Gewicht des Rohfaservlies ist und worin das Anstiegsverhältnis des Feuchtigkeitsaufnahmegleichgewichts des befeuchteten Faservlieses 0,5 bis 40 % im Vergleich zu dem Feuchtigkeitsaufnahmegleichgewicht des befeuchteten Faservlieses ist, nachdem die wasserlösliche Komponente entfernt ist, gemessen gemäß dem Verfahren nach JIS P8127 (Papier und Pappe - Bestimmung des Feuchtigkeitsgehaltes - Ofentrocknungsverfahren).

2. Das befeuchtete Faservlies gemäß Anspruch 1, worin der F5-Wert (Zugstärke, gemessen bei einer Verlängerung von 5 %) pro Einheit METSUKE (Gewicht pro Oberflächenflächeneinheit) 0,40 N·m$^2$/g oder weniger in der stärksten Richtung ist und die Stärke 1,0 N oder höher in der schwächsten Richtung ist.

3. Das befeuchtete Faservlies gemäß Anspruch 1, worin die Wasserabsorptionskapazität pro 1 g des befeuchteten Faservlieses nach 5 Sekunden vom Startpunkt der Messung durch das Larose-Verfahren 0,03 bis 2,50 ml/g ist und das Wasserrückhalteverhältnis 3,0 oder höher ist.

4. Das befeuchtete Faservlies gemäß Anspruch 1, worin der Biegewiderstand pro Einheit METSUKE (Gewicht pro Oberflächenflächeneinheit), gemessen durch ein Handle-O-Meter 5,0 mN·m$^2$/g oder weniger in der höchsten Biegungswiderstandsrichtung ist.

**5.** Das befeuchtete Faservlies gemäß Anspruch 1, worin der Biegewiderstand pro Einheit METSUKE (Gewicht pro Oberflächenflächeneinheit), gemessen durch das Ausleger-[Cantilever]-Verfahren 1,5 mm·m$^2$/g in der höchsten Biegungswiderstandsrichtung ist.

**6.** Das befeuchtete Faservlies gemäß Anspruch 1, worin der MIU-Wert (Reibungskoeffizientwert), gemessen durch einen Reibungssensitivitätstester 0,45 oder weniger ist.

**7.** Das befeuchtete Faservlies gemäß Anspruch 1, worin der Spitzenwert des Wärmeflusses Qmax 0,08 bis 0,30 J/cm$^2$/sek ist.

**8.** Das befeuchtete Faservlies gemäß Anspruch 1, bestehend aus mindestens einer von natürlichen Zellulosefasern, einschließend Zellstoffholz, regenerierten Fasern, einschließend Rayon-Fasern und synthetischen Fasern einschließend Polyethylenterephtalatfasern und Polypropylenfasern.

**9.** Das befeuchtete Faservlies gemäß Anspruch 1, bestehend aus einem Faservlies, erhalten durch Kombinieren von Fasernetzen, bestehend aus Fasern mit einer Länge von 20 bis 100 mm und Papierzellstoff, erhalten durch Nassvliesverfahren aus natürlichen Zellulosefasern mit einer Länge kürzer als 20 mm in einem Hydroverstrickungsverfahren.

**10.** Das befeuchtete Faservlies gemäß Anspruch 1, worin die wasserlösliche Komponente Glycerin umfasst.

**11.** Das befeuchtete Faservlies gemäß Anspruch 1, weiter umfassend eine zusätzliche Komponente, gewählt aus Ölen, Fettsäuren, höheren Alkoholen, Silikonen und Wachsen.

**Revendications**

**1.** Étoffe non-tissée humidifiée fabriquée en appliquant un liquide hydratant incluant un composant soluble dans l'eau, contenant un ou plusieurs composant(s) retenant l'humidité, choisis parmi la glycérine, la diglycérine, la polyglycérine, l'éthylène glycol, le diéthylène glycol, le polyéthylène glycol, le propylène glycol , le 1,3-butylène glycol, le sorbitol, le xylitol, l'érythritol, le mannitol, le lactitol, un alcool d'oligosaccharide, le maltitol, un produit d'hydrolyse d'amidon réduit, le fructose, le glucose, un oligosaccharide, le tréhalose, la glycine bétaïne, l'acide pyrrolidonecarboxylique, un sel d'acide pyrrolidone carboxylique, l'acide hyaluronique, un sel d'acide hyaluronique, l'acide lactique, un sel d'acide lactique, et l'urée, et de l'eau à une étoffe non-tissée brute par un traitement hydratant, dans laquelle l'étoffe non-tissée brute comprend 20 à 80% en poids de fibres d'une longueur de 20 à 100 mm et d'une finesse de 0,5 à 10,0 dtex et 20 à 80% en poids de fibres de cellulose naturelle d'une longueur plus courte que 20 mm et est lacée par filage, dans laquelle la quantité du composant soluble dans l'eau est de 5 à 150% par rapport au poids de l'étoffe non-tissée brute ; et dans laquelle le rapport augmentation de la reprise d'humidité à l'équilibre de l'étoffe non-tissée est de 0,5 à 40% par rapport à une reprise d'humidité à l'équilibre de l'étoffe non-tissée humidifiée après que le composant soluble dans l'eau soit enlevé, mesuré selon la procédure de la norme JIS P8127 (Papier et carton - Détermination de la teneur en humidité - Méthode par dessiccation à l'étuve).

**2.** Étoffe non-tissée humidifiée selon la revendication 1, dans laquelle la valeur F5 (résistance à la traction mesurée à un allongement de 5%) par unité Metsuke (poids par unité de surface) est de 0,40 N.m$^2$/g ou moins dans l'orientation la plus forte, et la force est de 1,0 N ou plus dans l'orientation la plus faible.

**3.** Étoffe non-tissée humidifiée selon la revendication 1, dans laquelle la capacité d'absorption d'eau pour 1 g de l'étoffe non-tissée humidifiée 5 secondes après le point de départ de la mesure par la méthode de Larose est de 0,03 à 2,50 ml/g, et le rapport de rétention d'eau est de 3,0 ou plus.

**4.** Étoffe non-tissée humidifiée selon la revendication 1, dans laquelle la résistance à la flexion par unité Metsuke (poids par unité de surface), mesurée à l'aide d'un Handle-O-meter, est de 5,0 mN.m$^2$/g ou moins dans le sens de résistance à la flexion le plus élevé.

**5.** Étoffe non-tissée humidifiée selon la revendication 1, dans laquelle la résistance à la flexion par unité Metsuke (poids par unité de surface), mesurée par la méthode cantilever, est de 1,5 mm.m$^2$/g dans le sens de résistance à la flexion le plus élevé.

6.  Étoffe non-tissée humidifiée selon la revendication 1, dans laquelle la valeur MIU (valeur du coefficient de frottement), mesurée à l'aide d'un testeur de sensibilité au frottement, est de 0,45 ou moins.

7.  Étoffe non-tissée humidifiée selon la revendication 1, dans laquelle la valeur de pic du flux de chaleur Qmax est de 0,08 à 0,30 J/cm²/sec.

8.  Étoffe non-tissée humidifiée selon la revendication 1, composée d'au moins l'une des fibres de cellulose naturelle incluant la pâte de bois, les fibres régénérées y compris les fibres de rayonne, et les fibres synthétiques incluant les fibres de téréphtalate de polyéthylène et les fibres de polypropylène.

9.  Étoffe non-tissée humidifiée selon la revendication 1, composée d'une étoffe non-tissée obtenue en combinant des voiles de fibres composés de fibres d'une longueur de 20 à 100 mm et de la pâte de papier obtenue par un procédé par voie humide à partir de fibres de cellulose naturelle d'une longueur inférieure à 20 mm dans un procédé d'hydro-emmêlement.

10. Étoffe non-tissée humidifiée selon la revendication 1, dans laquelle le composant soluble dans l'eau comprend de la glycérine.

11. Étoffe non-tissée humidifiée selon la revendication 1, comprenant en outre un composant additif choisi parmi les huiles, les acides gras, les alcools supérieurs, les silicones et les cires.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005287710 A
- WO 0064408 A
- EP 1222915 A
- US 20030114324 A
- US 20030049290 A
- JP 5156596 A
- JP 3578859 B
- EP 1306073 A